(19) Europäisches Patentamt / European Patent Office / Office européen des brevets

(11) **EP 2 897 576 B1**

(12) **EUROPÄISCHE PATENTSCHRIFT**

(45) Veröffentlichungstag und Bekanntmachung des Hinweises auf die Patenterteilung:
**01.03.2017 Patentblatt 2017/09**

(21) Anmeldenummer: **13760053.2**

(22) Anmeldetag: **13.09.2013**

(51) Int Cl.:
***A61K 8/36*** *(2006.01)* ***A61K 8/362*** *(2006.01)*
***A61K 8/365*** *(2006.01)* ***A61K 8/41*** *(2006.01)*
***A61K 8/898*** *(2006.01)* ***A61Q 5/06*** *(2006.01)*
***A61K 8/368*** *(2006.01)* ***A61K 8/55*** *(2006.01)*
***A61Q 5/10*** *(2006.01)*

(86) Internationale Anmeldenummer:
**PCT/EP2013/068973**

(87) Internationale Veröffentlichungsnummer:
**WO 2014/044602 (27.03.2014 Gazette 2014/13)**

(54) **MITTEL ZUM BEHANDELN KERATINISCHER FASERN, ENTHALTEND SPEZIFISCHE AMINOSILIKONE, SÄUREN UND DIREKTZIEHENDE FARBSTOFFE**

COMPOSITION FOR TREATING KERATINOUS FIBRES, COMPRISING SPECIFIC AMINOSILICONES, ACIDS AND DIRECT DYES

MOYEN DE TRAITEMENT DE FIBRES KÉRATINIQUES, CONTENANT DES AMINOSILICONES SPÉCIFIQUES, DES ACIDES ET DES COLORANTS DIRECTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.09.2012 DE 102012216606**

(43) Veröffentlichungstag der Anmeldung:
**29.07.2015 Patentblatt 2015/31**

(73) Patentinhaber: **Henkel AG & Co. KGaA**
**40589 Düsseldorf (DE)**

(72) Erfinder:
- **GOUTSIS, Konstantin**
  **41363 Jüchen (DE)**
- **WESER, Gabriele**
  **41472 Neuss (DE)**

(56) Entgegenhaltungen:
**WO-A1-2012/066722**
**US-A- 4 820 308**
**US-A1- 2003 147 841**
**US-A1- 2005 255 075**
**US-A1- 2009 074 699**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 897 576 B1

## Beschreibung

Die vorliegende Erfindung betrifft Mittel zum Behandeln von keratinischen Fasern, insbesondere menschlichen Haaren, die mindestens ein spezifisches Aminosilikon, mindestens eine anorganische und/oder organische Säure und mindestens einen direktziehenden Farbstoff enthalten. Weiterhin betrifft die vorliegende Anmeldung die Verwendung dieser speziellen Zusammensetzung zur Erzeugung von lang anhaltendem Glanz auf dem Haar und ein entsprechendes Verfahren.

Die Pflege und Behandlung des menschlichen Haares stellt einen wichtigen Bestandteil der täglichen Hygiene dar. Auch die Veränderung der Farbe des Haares ist ein zentraler Bereich der modernen Kosmetik. Die Behandlung mit Shampoos und Conditionern zählt zu den üblicherweise mehrmals die Woche, oft auch täglich angewandten Haarbehandlungsmitteln. Eine Veränderung der Haarfarbe erfolgt in der Regel in größeren Zeitabständen, ist für den Verbraucher aber im Hinblick auf das eigene Erscheinungsbild von ähnlich großer Bedeutung, da sie sowohl eine modische Ausdrucksform als auch eine Möglichkeit zur Kaschierung von grauen Haaren darstellt.

Besonders erstrebenswert für den Verbraucher sind Haarbehandlungsmittel, die dem Haar einen hohen Glanz verleihen. Glänzendes Haar wirkt attraktiv und gesund, die Frisur wird als gepflegt und vital wahrgenommen. Im Rahmen der Entwicklungsarbeiten von Haarglanz erzeugenden kosmetischen Mitteln wurden schon vielfache Bestrebungen unternommen, und eine entsprechend große Anzahl von Produkten befindet sich auf dem Markt.

Die WO 1997/038667 schlägt zur Erzielung eines natürlichen Glanzes bei gleichzeitiger guter Frisierbarkeit des Haares den Einsatz bestimmter fein dispergierter Partikel, beispielsweise dispergierten Silikonharzen, vor. Aus der DE 19757508 ist weiterhin bekannt, dass Haarbehandlungsmitteln zur Haarglanzverbesserung eine Substanz mit einem Brechungsindex oberhalb von 1,48 hinzugefügt werden kann. Nach Lehre der DE 102011089686 schließlich soll trockenem Haar mehr Glanz durch die Anwendung einer Kombination aus quartären Ammoniumverbindungen, Ölkörper, Kohlenwasserstoffen und zwei verschiedenen Silikonverbindungen verliehen werden.

US 4 820 308 betrifft kosmetische Zusammensetzungen, welche einen stabilen direktziehenden Farbstoff, ein Alkanolamin, ein kationisches Silikon sowie ein kationisches Tensid enthalten.

US 2005/255075 beschreibt kosmetische Mittel umfassend Amino-polyorganosiloxane, welche mit einer Alkylpolyglycolether-Gruppierung substituiert sind.

US 2009/0074699 A1 adressiert kosmetische Zusammensetzungen mit kationischem Copolymer und Aminosilikon.

WO 2012/066722 A1 beschreibt Shampoos enthaltend ein Aniontensid und ein aminosubstituiertes Organopolysiloxan.

In US 2003/0147841 A1 werden Aminosilikone in Kombination mit einem Reduktionsmittel eingesetzt. Trotz dieser umfangreichen Entwicklungsarbeiten ist es bislang jedoch nicht gelungen, Formulierungen zu finden, mittels welchen ein lang anhaltender Glanz erzeugt werden kann.

Bis dato zeigte das Haar direkt nach der Anwendung entsprechender Formulierungen zwar einen zufrieden stellenden Glanz, welcher jedoch nach der Wäsche des Haares - und spätestens nach einigen Wiederholungen der Haarwäsche - wieder verblasste, da bei der Wäsche auch die den Glanz erzeugenden Wirkstoffe wieder ausgespült wurden. Ein gegenüber mehrfachen Haarwäschen resistenter, lang anhaltender Glanz ist mit den Mitteln des Standes der Technik demnach nicht erzielbar.

Es war daher die Aufgabe der vorliegenden Anmeldung, ein Haarbehandlungsmittel bereitzustellen, mittels welchem auf den Haaren ein starker, natürlicher Glanz erzeugt werden kann, der lang anhaltend ist und auch nach wiederholten Haarwäschen nicht abnimmt. Auch nach mehrfacher Shampoobehandlung, soll das Haar möglichst noch genauso glänzen wie direkt nach der Behandlung mit dem Glanz erzeugenden Mittel.

In überraschender Weise konnte nun gefunden werden, dass kosmetische Mittel, die eine spezielle Kombination aus mindestens einer anorganischen und/oder organischen Säure, einem direktziehenden Farbstoff und einem spezifischen Aminosilikon enthalten, auf Haaren einen besonders lang anhaltenden Glanz erzeugen.

Ein erster Gegenstand der vorliegenden Erfindung ist daher ein Mittel zum Behandeln von keratinischen Fasern, insbesondere menschlichen Haaren, welches dadurch gekennzeichnet ist, dass es in einem kosmetisch geeigneten Träger

a) mindestens eine anorganische und/oder organische Säure
b) mindestens einen direktziehenden Farbstoff und
c) mindestens eine Verbindung der Formel (I)

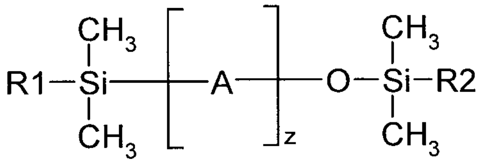

(I)

mit

A = 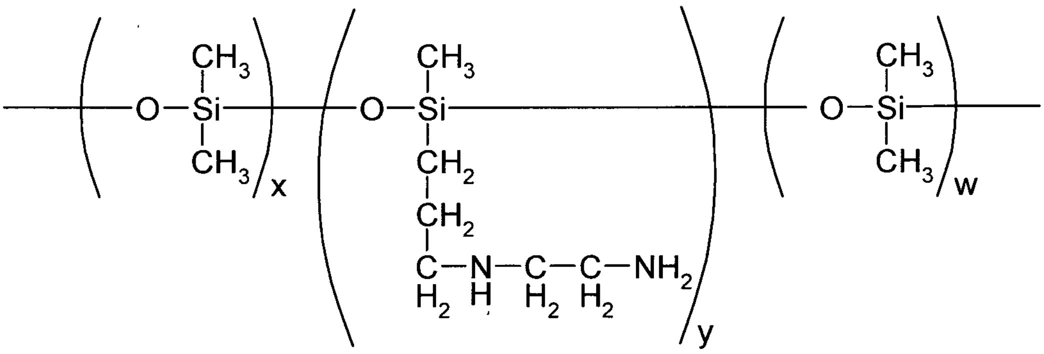

enthält, worin

i. x und y unabhängig von einander für Zahlen von 1 bis 100 stehen,
ii. w für eine Zahl von 0 bis 100 steht,
iii. z für eine Zahl von 1 bis 100 steht, wobei, falls $z \geq 2$ ist, die jeweiligen Werte x, y und w in einem Strukturelement A jeweils unabhängig von vorangehenden Strukturelementen A gewählt werden können und
iii. R1 und R2 unabhängig voneinander für eine lineare oder verzweigte, gesättigte, ungesättigte oder mehrfach ungesättigte $C_5$-$C_{20}$-Alkylgruppe stehen.

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Als lang anhaltender Glanz im Sinne der Erfindung ist der Glanz der keratinischen Fasern nach mehreren Haarwäschen, insbesondere nach 3 Haarwäschen, gemeint.

Die erfindungsgemäßen Mittel enthalten die Säure(n) a), den bzw. die direktziehenden Farbstoffe b) und die Verbindung(en) der Formel (I) c) in einem kosmetischen Träger, bevorzugt in einem geeigneten wässrigen, alkoholischen oder wässrig-alkoholischen Träger. Zum Zwecke der Haarbehandlung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die Inhaltsstoffe a), b) und c) in eine pulverförmige oder auch tablettenförmige Formulierung zu integrieren und diese vor der Anwendung mit einem wässrigen oder wässrig-alkoholischen Träger zu vermischen. Als ersten wesentlichen Formulierungsbestandteil enthält das erfindungsgemäße Mittel zur Behandlung von keratinischen Fasern mindestens eine anorganische und/oder organische Säure a).

Als Säuren kommen prinzipiell alle Verbindungen in Frage, die zur Abgabe eines Protons (einwertige Säure) oder mehrerer Protonen (mehrwertige Säure) in der Lage sind. Als anorganische Säuren können beispielsweise Mineralsäuren wie Salzsäure, Schwefelsäure und Phosphorsäure, bevorzugt in ihrer mit Wasser verdünnten Form, verwendet werden. Innerhalb der Gruppe der anorganischen Säuren sind die Schwefelsäure und die Phosphorsäure bevorzugt. Ganz besonders bevorzugt ist die Phosphorsäure. Auch organische Säuren können in den erfindungsgemäßen Formulierungen eingesetzt werden. Typische Vertreter für organische Säuren sind aliphatische Mono- und Dicarbonsäuren wie beispielsweise Essigsäure, Propionsäure, Oxalsäure und 1,3-Propandisäure sowie aromatische Carbonsäuren wie beispielsweise Benzoesäure. Weitere erfindungsgemäße organische Säuren sind Hydroxycarbonsäuren wie Glykolsäure, Zitronensäure, Weinsäure, Äpfelsäure und Milchsäure. Auch ungesättigte Mono- oder Dicarbonsäuren wie beispielsweise Fumarsäure oder $\alpha$-Ketocarbonsäuren wie beispielsweise Brenztraubensäure (2-Oxopropionsäure) sind erfindungsgemäß.

Zur Lösung der erfindungsgemäßen Aufgabenstellung können auch Mono- oder Diphosphonsäuren eingesetzt

werden. Ein Beispiel für eine bevorzugte Diphosphonsäure ist die 1-Hydroxyethan-1,1-diphosphonsäure. Heterozyklische Mono- und Dicarbonsäuren wie beispielsweise Pyridin-2-carbonsäure, Pyridin-3-carbonsäure, Pyridin-4-carbonsäure und Pyridin-2,6-dicarbonsäure zeigen ebenfalls sehr gute Eignung zur Erzeugung von Haarbehandlungsmitteln mit lang anhaltendem Glanz. Aufgrund der für kosmetische Mittel bestehenden formulierungstechnischen und gesetzlichen Anforderungen sind jedoch geruchsarme, für den Einsatz in Kosmetika zugelassene Säuren am besten geeignet zur Entwicklung von Haarbehandlungsmitteln mit lang anhaltendem Glanz. Haarbehandlungsmittel, die mindestens eine Säure ausgewählt aus Zitronensäure, Weinsäure, Äpfelsäure, Milchsäure, 1-Hydroxyethan-1,1-diphosphonsäure, 2,6-Dipicolinsäure und Benzoesäure enthalten, sind daher bevorzugt.

Eine bevorzugte Ausführungsform ist ein Mittel zum Behandeln von keratinischen Fasern, welches dadurch gekennzeichnet ist, dass es als anorganische und/oder organische Säure mindestens eine Säure ausgewählt aus Zitronensäure, Weinsäure, Äpfelsäure, Milchsäure, 1-Hydroxyethan-1,1-diphosphonsäure, 2,6-Dipicolinsäure und Benzoesäure enthält.

Innerhalb dieser Gruppe sind die Säuren Zitronensäure, Weinsäure, Äpfelsäure, und Milchsäure nochmals explizit ganz besonders gut geeignet. Enthält das erfindungsgemäße Mittel daher als Säure a) mindestens eine Säure ausgewählt aus Zitronensäure, Weinsäure, Äpfelsäure, und Milchsäure, so ist dies ganz besonders bevorzugt.

Die anorganischen und/oder organischen Säuren können - abhängig von ihrer Säurestärke und optional zusätzlich enthaltenden Puffersubstanzen oder alkalisch reagierenden Verbindungen - in einer Gesamtmenge von 0,001 bis 15 Gew.-%, bevorzugt 0,005 bis 12 Gew.-%, weiter bevorzugt 0,01 bis 9 Gew.-% und besonders bevorzugt von 0,1 bis 6 Gew.-% im Mittel enthalten sein, wobei sich die Gewichtsprozente jeweils auf die Gesamtmenge des Mittels beziehen.

Durch Abspaltung von Protonen erhöhen die Säuren in Anwesenheit von Wasser die Konzentration an $H^+$ bzw. $H3O^+$ Ionen, womit eine Absenkung des pH-Wertes verbunden ist. Im Zuge der zu dieser Erfindung führenden Arbeiten hat sich gezeigt, dass die Konzentration der im Mittel enthaltenen Säuren - und daraus resultierend auch der pH-Wert - die Stärke des erzeugbaren Glanzes ganz wesentlich beeinflusst. Die keratinischen Fasern glänzen insbesondere dann stark und lang anhaltend, wenn das auf ihnen angewendete erfindungsgemäße Mittel einen pH-Wert von kleiner als 7 besitzt.

Vorteilhaft ist es deshalb, wenn das erfindungsgemäße Mittel auf einen pH-Wert von 2 bis 7, bevorzugt von 3 bis 6,9, weiter bevorzugt von 4 bis 6,8 und insbesondere bevorzugt von 5 bis 6,7 besitzt.

Bei den im Sinne dieser Erfindung gemessenen pH-Werten handelt es sich um pH-Werte, die bei einer Temperatur von 22 °C gemessen wurden. Zur Messung des pH-Wertes eignen sich insbesondere Glaselektroden.

Eine weitere bevorzugte Ausführungsform ist daher ein Mittel zum Behandeln von keratinischen Fasern, welches dadurch gekennzeichnet ist, dass es bei einer Temperatur von 22 °C einen pH-Wert von 2 bis 7, bevorzugt von 3 bis 6,9 , weiter bevorzugt von 4 bis 6,8 und insbesondere bevorzugt von 5 bis 6,7 besitzt.

Zur Stabilisierung des jeweiligen gewünschten pH-Wertes können die erfindungsgemäßen Mittel zusätzlich auch Puffer bzw. Puffersysteme enthalten. Definitionsgemäß sind Puffersysteme Lösungen einer schwachen Säure mit einem praktisch völlig dissoziierten Salz derselben Säure.

Erfindungsgemäße Puffer können sich zusammensetzen aus einer schwachen anorganischen und/oder organischen Säure a), die im Gemisch mit ihrem Salz eingesetzt wird. Es ist aber ebenfalls möglich, dass das erfindungsgemäße Mittel eine starke anorganischen und/oder organischen Säure a) enthält und dem Mittel als Puffer zusätzlich das Gemisch einer schwachen Säure und ihres Salzes zugesetzt ist. Bei den Salzen der schwachen Säuren kann es sich um ihre Alkalisalze, bevorzugt um ihre Natrium- und Kaliumsalze, handeln.

Erfindungsgemäße Puffersysteme sind beispielsweise Essigsäure/Natriumacetat, Essigsäure /Kaliumacetat, Zitronensäure/Natriumzitrat, Zitronensäure/Kaliumzitrat, Weinsäure/Natriumtartrat, Weinsäure/Kaliumtartrat, Milchsäure/Natriumlactat und Milchsäure/Kaliumlactat.

Als zweiten wesentlichen Inhaltsstoff enthält das erfindungsgemäße Mittel zur Behandlung von keratinischen Fasern mindestens einen direktziehenden Farbstoff b). Bei direktziehenden Farbstoffen handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden. Die direktziehenden Farbstoffe können in einer Gesamtmenge von 0,0001 bis 5 Gew.-%, bevorzugt 0,001 bis 2,5 Gew.-%, weiter bevorzugt 0,01 bis 1 Gew.-% und insbesondere bevorzugt 0,02 bis 0,25 Gew.-%, bezogen auf das Gesamtgewicht des Mittels, im Mittel enthalten sein.

In einer besonders bevorzugten Ausführungsform führen die im erfindungsgemäßen Mittel enthaltenen direktziehenden Farbstoffe nicht zu einer starken Färbung der keratinischen Fasern, sondern lediglich zu einer den Glanz unterstützenden, leichten Nuancierung. In diesem Fall beträgt die Gesamtmenge aller im erfindungsgemäßen Mittel enthaltenen direktziehenden Farbstoffe weniger als 1 Gew.-%, bevorzugt weniger als 0,5 Gew.-% und insbesondere bevorzugt weniger als 0,25 Gew.-%, bezogen auf das Gesamtgewicht des Mittels.

Eine weitere bevorzugte Ausführungsform ist daher ein Mittel zum Behandeln von keratinischen Fasern, welches dadurch gekennzeichnet ist, dass es einen oder mehrere direktziehende Farbstoffe in einer Gesamtmenge von 0,0001 bis 5 Gew.-%, bevorzugt 0,001 bis 2,5 Gew.-%, weiter bevorzugt 0,01 bis 1 Gew.-% und insbesondere bevorzugt 0,02 bis 0,25 Gew.-%, bezogen auf das Gesamtgewicht des Mittels, enthält.

Geeignete anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Geeignete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe.

Als nichtionische direktziehende Farbstoffe eignen sich insbesondere nichtionische Nitro- und Chinonfarbstoffe und neutrale Azofarbstoffe. Geeignete nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethyl-amino-3-nitrobenzoesäure und 2-Chlor-6-ethylamino-4-nitrophenol bekannten Verbindungen.

In den erfindungemäßen Mitteln können alle denkbaren Kombinationen der oben genannten direktziehenden Farbstoffe enthalten sein. Bestimmte Farbstoffkombinationen haben sich im Hinblick auf eine dauerhafte Glanzerzeugung jedoch als besonders vorteilhaft erwiesen.

Bevorzugt enthält das erfindungsgemäße Mittel als direktziehenden Farbstoff b) einen nichtionischen Farbstoff. Ganz besonders bevorzugt enthält das erfindungsgemäße Mittel als direktziehenden Farbstoff b) einen nichtionischen Nitrofarbstoff.

Entsprechend sind die Mittel besonders bevorzugt, welche

a) mindestens eine anorganische und/oder organische Säure
b) mindestens einen direktziehenden Farbstoff aus der Gruppe der nichtionischen Nitrofarbstoffe und
c) mindestens eine Verbindung der Formel (I) enthalten.

Eine weitere bevorzugte Ausführungsform ist daher ein Mittel zum Behandeln von keratinischen Fasern, welches dadurch gekennzeichnet ist, dass es als direktziehenden Farbstoff mindestens einen nichtionischen Nitrofarbstoff enthält.

Bei nichtionischen Nitrofarbstoffen handelt es sich definitionsgemäß um substituierte Nitrobenzolderivate. Diese Nitrobenzolderivate können beispielsweise mit einen oder mehreren Substituenten ausgewählt aus einer Aminogruppe, einer Hydroxygruppe, einer $C_1$-$C_6$-Alkylgruppe, einer $C_1$-$C_6$-Alkylaminogruppe, einer $C_1$-$C_6$-Dialkylaminogruppe, einer $C_1$-$C_6$-Alkoxygruppe, einem Halogenatom, einer Hydroxy-$C_2$-$C_6$-alkylaminogruppe oder einer Bis(hydroxy-C2-C6-alkyl)aminogruppe substituiert sein.

Weiterhin haben sich spezielle Kombinationen nichtionischer Nitrofarbstoffe sich im Hinblick auf die Erzeugung eines optimalen Farb- und Glanzresultats als besonders geeignet erwiesen.

Eine weitere bevorzugte Ausführungsform ist daher ein Mittel, welches eine der folgenden Kombinationen an direktziehenden Farbstoffen enthält: HC Blue 12/ HC Yellow 2, HC Blue 12/ HC Yellow 4, HC Blue 12/ HC Yellow 5, HC Blue 12/ HC Yellow 6, HC Blue 12/ HC Yellow 12, HC Blue 12/ HC Orange 1, HC Blue 12/ HC Red 1, HC Blue 12/ HC Red 3, HC Blue 12/ HC Red 7, HC Blue 12/ HC Red 10, HC Blue 12/ HC Red 11, HC Blue 12/ HC Red 13, HC Blue 12/ HC Red BN, HC Blue 12/ HC Violet 1, HC Blue 12/1,4-Diamino-2-nitrobenzol, HC Blue 12/2-Amino-4-nitrophenol, HC Blue 12/1,4-Bis-(2-hydroxyethyl)amino-2-nitrobenzol, HC Blue 12/3-Nitro-4-(2-hydroxyethyl)aminophenol, HC Blue 12/4-[(2-Hydroxyethyl)amino]-3-nitro-1-methylbenzol, HC Blue 12/1-Amino-4-(2-hydroxyethyl)amino-5-chlor-2-nitrobenzol, HC Blue 12/1-Amino-4-(2-hydroxyethyl)amino-5-chlor-2-nitrobenzol, HC Blue 12/4-Amino-3-nitrophenol, HC Blue 12/4-[(3-Hydroxypropyl)amino]-3-nitrophenol, HC Blue 12/2-Amino-6-chloro-4-nitrophenol, HC Blue 12/4-Ethylamino-3-nitrobenzoesäure oder HC Blue 12/2-Chlor-6-ethylamino-4-nitrophenol.

Eine ebenfalls bevorzugte Ausführungsform ist ein Mittel, welches eine der folgenden Kombinationen an direktziehenden Farbstoffen enthält: HC Yellow 2/ HC Yellow 4, HC Yellow 2/ HC Yellow 5, HC Yellow 2/ HC Yellow 6, HC Yellow 2/ HC Yellow 12, HC Yellow 2/ HC Orange 1, HC Yellow 2/ HC Red 1, HC Yellow 2/ HC Red 3, HC Yellow 2/ HC Red 7, HC Yellow 2/ HC Red 10, HC Yellow 2/ HC Red 11, HC Yellow 2/ HC Red 13, HC Yellow 2/ HC Red BN, HC Yellow 2/HC Violet 1, HC Yellow 2 /HC Blue 2, HC Yellow 2/ HC Blue 11, HC Yellow 2/HC Blue 12, HC Yellow 2/1,4-Diamino-2-nitrobenzol, HC Yellow 2/2-Amino-4-nitrophenol, HC Yellow 2/1,4-Bis-(2-hydroxyethyl)amino-2-nitrobenzol, HC Yellow 2/3-Nitro-4-(2-hydroxyethyl)aminophenol, HC Yellow 2/4-[(2-Hydroxyethyl)amino]-3-nitro-1-methylbenzol, HC Yellow 2/1-Amino-4-(2-hydroxyethyl)amino-5-chlor-2-nitrobenzol, HC Yellow 2/4-Amino-3-nitrophenol, HC Yellow 2/4-[(3-Hydroxypropyl)amino]-3-nitrophenol, HC Yellow 2/2-Amino-6-chloro-4-nitrophenol, HC Yellow 2/4-Ethylamino-3-nitrobenzoesäure oder HC Yellow 2/2-Chlor-6-ethylamino-4-nitrophenol.

Eine ebenfalls bevorzugte Ausführungsform ist ein Mittel, welches eine der folgenden Kombinationen an direktziehenden Farbstoffen enthält: 2-Amino-6-chloro-4-nitrophenol/HC Yellow 2, 2-Amino-6-chlor-4-nitrophenol/ HC Yellow 4, 2-Amino-6-chlor-4-nitrophenol/ HC Yellow 5, 2-Amino-6-chlor-4-nitrophenol/ HC Yellow 6, 2-Amino-6-chlor-4-nitrophenol/ HC Yellow 12, 2-Amino-6-chlor-4-nitrophenol/ HC Orange 1, 2-Amino-6-chlor-4-nitrophenol/ HC Red 1, 2-Amino-6-chlor-4-nitrophenol/ HC Red 3, 2-Amino-6-chlor-4-nitrophenol/ HC Red 7, 2-Amino-6-chlor-4-nitrophenol/ HC Red 10, 2-Amino-6-chlor-4-nitrophenol/ HC Red 11, 2-Amino-6-chlor-4-nitrophenol/ HC Red 13, 2-Amino-6-chlor-4-nitrophenol/ HC Red BN, 2-Amino-6-chlor-4-nitrophenol/ HC Blue 2, 2-Amino-6-chlor-4-nitrophenol/ HC Blue 11 2-Amino-6-chlor-4-nitrophenol/ HC Blue 12, 2-Amino-6-chlor-4-nitrophenol/HC Violet 1, 2-Amino-6-chlor-4-nitrophenol/1,4-Diamino-2-nitrobenzol, 2-Amino-6-chlor-4-nitrophenol/2-Amino-4-nitrophenol, 2-Amino-6-chlor-4-nitrophenol/1,4-Bis-(2-hydroxyethyl)amino-2-nitrobenzol, 2-Amino-6-chlor-4-nitrophenol/3-Nitro-4-(2-hydroxyethyl)aminophenol, 2-Amino-6-chlor-4-nitrophenol/4-[(2-Hydroxyethyl)-amino]-3-nitro-1-methylbenzol, 2-Amino-6-chlor-4-nitrophenol/1-Amino-4-(2-hydroxyethyl)amino-5-chlor-2-nitrobenzol, 2-Amino-6-chlor-4-nitrophenol/4-Amino-3-nitrophenol, 2-Amino-6-chlor-4-nitrophenol/4-[(3-Hydroxypropyl)amino]-3-nitrophenol, 2-Amino-6-chlor-4-nitrophenol/4-Ethylamino-3-nitrobenzoesäure oder 2-Amino-6-chlor-4-nitrophenol/2-Chlor-6-ethylamino-4-nitrophenol.

Eine weitere bevorzugte Ausführungsform ist daher ein Mittel zum Behandeln von keratinischen Fasern, welches dadurch gekennzeichnet ist, dass es eine der folgenden Kombinationen an direktziehenden Farbstoffen enthält: HC Blue 12/ HC Yellow 2, HC Blue 12/ HC Yellow 4, HC Blue 12/ HC Yellow 5, HC Blue 12/ HC Yellow 6, HC Blue 12/ HC Yellow 12, HC Blue 12/ HC Orange 1, HC Blue 12/ HC Red 1, HC Blue 12/ HC Red 3, HC Blue 12/ HC Red 7, HC Blue 12/ HC Red 10, HC Blue 12/ HC Red 11, HC Blue 12/ HC Red 13, HC Blue 12/ HC Red BN, HC Blue 12/ HC Violet 1, HC Blue 12/1,4-Diamino-2-nitrobenzol, HC Blue 12/2-Amino-4-nitrophenol, HC Blue 12/1,4-Bis-(2-hydroxyethyl)amino-2-nitrobenzol, HC Blue 12/3-Nitro-4-(2-hydroxyethyl)aminophenol, HC Blue 12/4-[(2-Hydroxyethyl)amino]-3-nitro-1-methylbenzol, HC Blue 12/1-Amino-4-(2-hydroxyethyl)amino-5-chlor-2-nitrobenzol, HC Blue 12/1-Amino-4-(2-hydroxyethyl)amino-5-chlor-2-nitrobenzol, HC Blue 12/4-Amino-3-nitrophenol, HC Blue 12/4-[(3-Hydroxypropyl)amino]-3-nitrophenol, HC Blue 12/2-Amino-6-chloro-4-nitrophenol, HC Blue 12/4-Ethylamino-3-nitrobenzoesäure, HC Blue 12/2-Chlor-6-ethylamino-4-nitrophenol, HC Yellow 2/ HC Yellow 4, HC Yellow 2/ HC Yellow 5, HC Yellow 2/ HC Yellow 6, HC Yellow 2/ HC Yellow 12, HC Yellow 2/ HC Orange 1, HC Yellow 2/ HC Red 1, HC Yellow 2/ HC Red 3, HC Yellow 2/ HC Red 7, HC Yellow 2/ HC Red 10, HC Yellow 2/ HC Red 11, HC Yellow 2/ HC Red 13, HC Yellow 2/ HC Red BN, HC Yellow 2/HC Violet 1, HC Yellow 2 /HC Blue 2, HC Yellow 2/ HC Blue 11, HC Yellow 2/HC Blue 12, HC Yellow 2/1,4-Diamino-2-nitrobenzol, HC Yellow 2/2-Amino-4-nitrophenol, HC Yellow 2/1,4-Bis-(2-hydroxyethyl)amino-2-nitrobenzol, HC Yellow 2/3-Nitro-4-(2-hydroxyethyl)aminophenol, HC Yellow 2/4-[(2-Hydroxyethyl)amino]-3-nitro-1-methylbenzol, HC Yellow 2/1-Amino-4-(2-hydroxyethyl)amino-5-chlor-2-nitrobenzol, HC Yellow 2/4-Amino-3-nitrophenol, HC Yellow 2/4-[(3-Hydroxypropyl)amino]-3-nitrophenol, HC Yellow 2/2-Amino-6-chloro-4-nitrophenol, HC Yellow 2/4-Ethylamino-3-nitrobenzoesäure oder HC Yellow 2/2-Chlor-6-ethylamino-4-nitrophenol, 2-Amino-6-chloro-4-nitrophenol/HC Yellow 2, 2-Amino-6-chlor-4-nitrophenol/ HC Yellow 4, 2-Amino-6-chlor-4-nitrophenol/ HC Yellow 5, 2-Amino-6-chlor-4-nitrophenol/ HC Yellow 6, 2-Amino-6-chlor-4-nitrophenol/ HC Yellow 12, 2-Amino-6-chlor-4-nitrophenol/ HC Orange 1, 2-Amino-6-chlor-4-nitrophenol/ HC Red 1, 2-Amino-6-chlor-4-nitrophenol/ HC Red 3, 2-Amino-6-chlor-4-nitrophenol/ HC Red 7, 2-Amino-6-chlor-4-nitrophenol/ HC Red 10, 2-Amino-6-chlor-4-nitrophenol/ HC Red 11, 2-Amino-6-chlor-4-nitrophenol/ HC Red 13, 2-Amino-6-chlor-4-nitrophenol/ HC Red BN, 2-Amino-6-chlor-4-nitrophenol/ HC Blue 2, 2-Amino-6-chlor-4-nitrophenol/ HC Blue 11, 2-Amino-6-chlor-4-nitrophenol/ HC Blue 12, 2-Amino-6-chlor-4-nitrophenol/HC Violet 1, 2-Amino-6-chlor-4-nitrophenol/1,4-Diamino-2-nitrobenzol, 2-Amino-6-chlor-4-nitrophenol/2-Amino-4-nitrophenol, 2-Amino-6-chlor-4-nitrophenol/1,4-Bis-(2-hydroxyethyl)amino-2-nitrobenzol, 2-Amino-6-chlor-4-nitrophenol/3-Nitro-4-(2-hydroxyethyl)aminophenol, 2-Amino-6-chlor-4-nitrophenol/4-[(2-Hydroxyethyl)amino]-3-nitro-1-methylbenzol, 2-Amino-6-chlor-4-nitrophenol/1-Amino-4-(2-hydroxyethyl)amino-5-chlor-2-nitrobenzol, 2-Amino-6-chlor-4-nitrophenol/4-Amino-3-nitrophenol, 2-Amino-6-chlor-4-nitrophenol/4-[(3-Hydroxypropyl)amino]-3-nitrophenol, 2-Amino-6-chlor-4-nitrophenol/4-Ethylamino-3-nitrobenzoesäure oder 2-Amino-6-chlor-4-nitrophenol/2-Chlor-6-ethylamino-4-nitrophenol. Explizit ganz besonders bevorzugt ist es, wenn das erfindungsgemäße Mittel eine Kombination aus den 4 direktziehenden, nichtionischen Farbstoffen HC Yellow 2, HC Blue 12, 2-Amino-6-chlor-4-nitrophenol und N,N-Bis(2-hydroxyethyl)-2-nitro-p-phenlyendiamin enthält.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als dritten wesentlichen Fomulierungsbestandteil c) enthält das erfindungsgemäße Mittel zur Behandlung von keratinischen Fasern mindestens eine Verbindung der Formel (I)

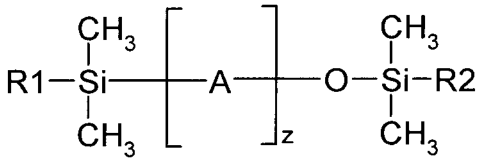

(I)

mit

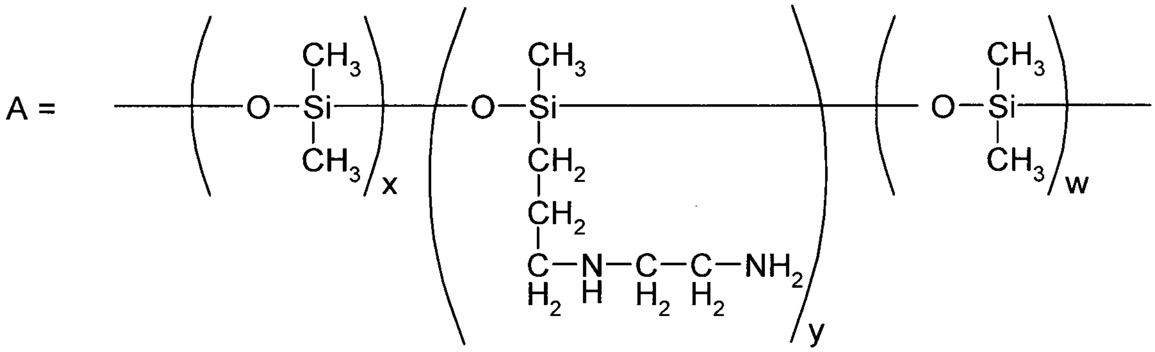

worin

i. x und y unabhängig von einander für Zahlen von 1 bis 100 stehen,
ii. w für eine Zahl von 0 bis 100 steht,
iii. z für eine Zahl von 1 bis 100 steht, wobei, falls z ≥ 2 ist, die jeweiligen Werte x, y und w in einem Strukturelement A jeweils unabhängig von vorangehenden Strukturelementen A gewählt werden können
und
iii. R1 und R2 unabhängig voneinander für eine lineare oder verzweigte, gesättigte, ungesättigte oder mehrfach ungesättigte $C_5$-$C_{20}$-Alkylgruppe stehen.

Die Strukturelemente A der Verbindung der Formel (I) sind erfindungsgemäß aus je einem oder mehreren Elementen {3-[(2-Aminoethyl)amino]propyl}{methyl}siloxan und einem oder mehreren Elementen Dimethylsiloxan aufgebaut. Die Anzahl an Dimethylsiloxan Elementen wird durch die Parameter x und w definiert. Die Anzahl an {3-[(2-Aminoethyl)amino]propyl}{methyl}siloxan Elementen wird durch den Parameter y definiert. Die Werte der Parameter x und y stehen erfindungsgemäß unabhängig voneinander für Zahlen zwischen 1 und 100, der Parameter w kann erfindungsgemäß für eine Zahl von 0 bis 100 stehen.

Die Anzahl an Strukturelementen A wird durch den Parameter z vorgegeben. Erfindungsgemäß liegt der Wert des Parameters z zwischen 1 und 100. Falls z ≥ 2, können die Parameter x, y und w in jedem Strukturelement A unabhängig von vorangehenden Strukturelementen A gewählt werden. Daraus folgt, dass sich für den Fall z ≥ 2 die einzelnen Strukturelemente A in ihrer Anzahl an {3-[(2-Aminoethyl)amino]propyl}{methyl}siloxan Elementen und/oder in ihrer Anzahl an Dimethylsiloxan Elementen voneinander unterscheiden können.

Das Siloxan-Grundgerüst der Verbindung(en) der Formel (I) wird gemäß der vorliegenden Erfindung an beiden Enden von den Resten R1 und R2 terminiert, wobei R1 und R2 unabhängig voneinander für eine lineare oder verzweigte, gesättigte, ungesättigte oder mehrfach ungesättigte $C_5$-$C_{20}$-Alkylgruppe stehen. R1 und R2 stehen für einen verzweigten, gesättigten, ungesättigten oder mehrfach ungesättigten $C_5$-$C_{20}$-Alkylrest, das Siloxan-Gerüst ist mit einer Fettalkylkette terminiert. Fettalkylketten im Sinne der vorliegenden Erfindung sind alle linearen und/oder verzweigten, gesättigten und/oder ungesättigten und/oder mehrfach ungesättigten Kohlenstoffketten, deren Kohlenstoffkette bevorzugt eine $C_6$-$C_{30}$-Kette besonders bevorzugt eine $C_8$-$C_{24}$-Kette und insbesondere eine $C_{14}$-$C_{20}$-Kette ist. Beispiele für erfindungsgemäße Fettalkylketten sind Hexyl, Heptyl, Octyl, Nonyl, Decyl, Dodecyl, Tetradeyl, Pentadecyl, Hexadecyl, Heptadecyl,

Octadecyl, Nonadecyl, Eicosyl, Docosyl, Tetracosyl, Hexacosyl, iso-Stearyl, (9Z)- Tetradeca- 9-enyl, (9Z)- Hexadeca-9-enyl, (6Z)- Octadeca- 6-enyl, (9Z)- Octadeca- 9-enyl, (9E)- Octadeca- 9-enyl, (11E)- Octadeca-11-enyl, (9Z)- Eicosa-9-enyl, (11Z)- Eicosa-11-enyl, (11Z)- Docosa-11-enyl, (13Z)- Docosa- 13-enyl, (15Z)- Tetracosa-15- enyl, (9Z,12Z)-Octadeca-9,12-dienyl, (9Z,12Z,15Z)- Octadeca- 9,12,15-trienyl, (6Z,9Z,12Z)- Octadeca- 6,9,12- trienyl, (8E,10E,12Z)-Octadeca- 8,10,12- trienyl, (9Z,11E,13Z)- Octadeca- 9,11,13-trienyl, (9Z,11E,13E)-Octadeca- 9,11,13-trienyl, (9E,11E,13E)-Octadeca- 9,11,13-trienyl, (5Z,8Z,11Z,14Z)- Eicosa- 5,8,11,14- tetraenyl, (5Z,8Z,11Z,14Z,17Z)-Eicosa-5,8,11,14,17-pentaenyl, (7Z,10Z,13Z,16Z,19Z)- Docosa- 7,10,13,16,19-pentaenyl und (4Z,7Z,10Z,13Z,16Z,19Z)-Docosa- 4,7,10,13,16,19- hexaenyl.

In einer bevorzugten Ausführungsform der Erfindung stehen die Reste R1 und R2 unabhängig voneinander für lineare Alkylketten, bevorzugt $C_{14}$-$C_{20}$-Alkyl, besonders bevorzugt Tetradeyl, Pentadecyl, Hexadecyl, Heptadecyl, Octadecyl, Nonadecyl oder Eicosyl. Insbesondere bevorzugt sind Hexadecyl (Cetyl) und/oder Octadecyl (Stearyl). Unter Cetearyl versteht sich eine Mischung aus Cetyl und Stearyl, diese Mischung ist ebenfalls bevorzugt.

In einer weiteren bevorzugten Ausführungsform ist ein erfindungsgemäßes Mittel daher dadurch gekennzeichnet, dass das erfindungsgemäße Mittel eine Verbindung der Formel (I) enthält, in der die Substituenten R1 und R2 unabhängig voneinander für eine lineare oder verzweigte, gesättigte, ungesättigte oder mehrfach ungesättigte $C_5$-$C_{20}$-Alkylkette, bevorzugt für eine lineare $C_{14}$-$C_{20}$-Alkylkette, insbesondere bevorzugt für einen Vertreter aus der Gruppe $H_3C$-$(CH_2)_{13}$-, $H_3C$-$(CH_2)_{15}$-, $H_3C$-$(CH_2)_{17}$-, $H_3C$-$(CH_2)_{19}$-, stehen.

Erfindungsgemäß besonders bevorzugt sind Verbindungen der Formel (I), bei denen die Reste R1 und R2 unabhängig voneinander für $H_3C$-$(CH_2)_{15}$- oder $H_3C$-$(CH_2)_{17}$-. In diesem Fall handelt es sich bei dem erfindungsgemäßen Amodimethicon um ein Bis-Cetearyl Amodimethicon.

Ganz besonders sind die Mittel besonders bevorzugt, welche

a) mindestens eine anorganische und/oder organische Säure
b) mindestens einen direktziehenden Farbstoff aus der Gruppe der nichtionischen Nitrofarbstoffe und
c) mindestens ein Bis-Cetearyl Amodimethicon enthalten.

In einerweiteren bevorzugten Ausführungsform ist ein erfindungsgemäßes Mittel daher dadurch gekennzeichnet, dass es eine Verbindung der Formel (I) enthält, bei welcher R1 für $H_3C$-$(CH_2)_{15}$- oder $H_3C$-$(CH_2)_{17}$- und R2 für $H_3C$-$(CH_2)_{15}$- oder $H_3C$-$(CH_2)_{17}$- steht. Solche Verbindungen sind unter der INCI-Bezeichnung Bis-Cetearyl Amodimethicon bekannt.

Die Verbindung(en) der Formel (I) können in dem erfindungsgemäßen Mittel in einer Gesamtmenge von 0,005 bis 5 Gew.-%, bevorzugt 0,1 bis 4,5 Gew.-%, besonders bevorzugt 1 bis 3 Gew.-% und insbesondere bevorzugt 1,5 bis 2,5 Gew.-%, bezogen auf das Gesamtgewicht des Mittels, enthalten sein.

In einer weiteren Ausführungsform ist das erfindungsgemäße Mittel dadurch gekennzeichnet, dass es die Verbindung bzw. die Verbindungen der Formel (I) in einer Gesamtmenge von 0,005 bis 5 Gew.-%, bevorzugt 0,1 bis 4,5 Gew.-%, besonders bevorzugt 1 bis 3 Gew.-% und insbesondere bevorzugt 1,5 bis 2,5 Gew.-%, bezogen auf das Gesamtgewicht des Mittels, enthält.

Die Zusammensetzung einiger bevorzugter erfindungsgemäßer Haarbehandlungsmittel können den folgenden Tabellen entnommen werden (alle Angaben sind in Gew.-%, bezogen auf das Gesamtgewicht des Mittels):

| Nr. | a) anorganische / organische Säure | b) direktziehender Farbstoff | c) Verbindung(en) der Formel (I) | pH-Wert des Mittels |
|---|---|---|---|---|
| 1 | Zitronensäure | 0,0001 bis 5 Gew.-% | 0,005 bis 5 Gew.-% | 7,0 - 6,5 |
| 2 | Zitronensäure | 0,001 bis 2,5 Gew.-% | 0,005 bis 5 Gew.-% | 7,0 - 6,5 |
| 3 | Zitronensäure | 0,01 bis 1 Gew.-% | 0,005 bis 5 Gew.-% | 7,0 - 6,5 |
| 4 | Zitronensäure | 0,02 bis 0,25 Gew.-% | 0,005 bis 5 Gew.-% | 7,0 - 6,5 |
| 5 | Zitronensäure | 0,0001 bis 5 Gew.-% | 0,005 bis 5 Gew.-% | 6,5 - 6,0 |
| 6 | Zitronensäure | 0,001 bis 2,5 Gew.-% | 0,005 bis 5 Gew.-% | 6,5 - 6,0 |
| 7 | Zitronensäure | 0,01 bis 1 Gew.-% | 0,005 bis 5 Gew.-% | 6,5 - 6,0 |
| 8 | Zitronensäure | 0,02 bis 0,25 Gew.-% | 0,005 bis 5 Gew.-% | 6,5 - 6,0 |
| 9 | Zitronensäure | 0,0001 bis 5 Gew.-% | 0,005 bis 5 Gew.-% | 6,0 - 5,5 |
| 10 | Zitronensäure | 0,001 bis 2,5 Gew.-% | 0,005 bis 5 Gew.-% | 6,0 - 5,5 |

(fortgesetzt)

| Nr. | a) anorganische / organische Säure | b) direktziehender Farbstoff | c) Verbindung(en) der Formel (I) | pH-Wert des Mittels |
|---|---|---|---|---|
| 11 | Zitronensäure | 0,01 bis 1 Gew.-% | 0,005 bis 5 Gew.-% | 6,0 - 5,5 |
| 12 | Zitronensäure | 0,02 bis 0,25 Gew.-% | 0,005 bis 5 Gew.-% | 6,0 - 5,5 |
| 13 | Zitronensäure | 0,0001 bis 5 Gew.-% | 0,005 bis 5 Gew.-% | 5,5 - 5,0 |
| 14 | Zitronensäure | 0,001 bis 2,5 Gew.-% | 0,005 bis 5 Gew.-% | 5,5 - 5,0 |
| 15 | Zitronensäure | 0,01 bis 1 Gew.-% | 0,005 bis 5 Gew.-% | 5,5 - 5,0 |
| 16 | Zitronensäure | 0,02 bis 0,25 Gew.-% | 0,005 bis 5 Gew.-% | 5,5 - 5,0 |
| 17 | Weinsäure | 0,0001 bis 5 Gew.-% | 0,005 bis 5 Gew.-% | 7,0 - 6,5 |
| 18 | Weinsäure | 0,001 bis 2,5 Gew.-% | 0,005 bis 5 Gew.-% | 7,0 - 6,5 |
| 19 | Weinsäure | 0,01 bis 1 Gew.-% | 0,005 bis 5 Gew.-% | 7,0 - 6,5 |
| 20 | Weinsäure | 0,02 bis 0,25 Gew.-% | 0,005 bis 5 Gew.-% | 7,0 - 6,5 |
| 21 | Weinsäure | 0,0001 bis 5 Gew.-% | 0,005 bis 5 Gew.-% | 6,5 - 6,0 |
| 22 | Weinsäure | 0,001 bis 2,5 Gew.-% | 0,005 bis 5 Gew.-% | 6,5 - 6,0 |
| 23 | Weinsäure | 0,01 bis 1 Gew.-% | 0,005 bis 5 Gew.-% | 6,5 - 6,0 |
| 24 | Weinsäure | 0,02 bis 0,25 Gew.-% | 0,005 bis 5 Gew.-% | 6,5 - 6,0 |
| 25 | Weinsäure | 0,0001 bis 5 Gew.-% | 0,005 bis 5 Gew.-% | 6,0 - 5,5 |
| 26 | Weinsäure | 0,001 bis 2,5 Gew.-% | 0,005 bis 5 Gew.-% | 6,0 - 5,5 |
| 27 | Weinsäure | 0,01 bis 1 Gew.-% | 0,005 bis 5 Gew.-% | 6,0 - 5,5 |
| 28 | Weinsäure | 0,02 bis 0,25 Gew.-% | 0,005 bis 5 Gew.-% | 6,0 - 5,5 |
| 29 | Weinsäure | 0,0001 bis 5 Gew.-% | 0,005 bis 5 Gew.-% | 5,5 - 5,0 |
| 30 | Weinsäure | 0,001 bis 2,5 Gew.-% | 0,005 bis 5 Gew.-% | 5,5 - 5,0 |
| 31 | Weinsäure | 0,01 bis 1 Gew.-% | 0,005 bis 5 Gew.-% | 5,5 - 5,0 |
| 32 | Weinsäure | 0,02 bis 0,25 Gew.-% | 0,005 bis 5 Gew.-% | 5,5 - 5,0 |
| 33 | Äpfelsäure | 0,0001 bis 5 Gew.-% | 0,005 bis 5 Gew.-% | 7,0 - 6,5 |
| 34 | Äpfelsäure | 0,001 bis 2,5 Gew.-% | 0,005 bis 5 Gew.-% | 7,0 - 6,5 |
| 35 | Äpfelsäure | 0,01 bis 1 Gew.-% | 0,005 bis 5 Gew.-% | 7,0 - 6,5 |
| 36 | Äpfelsäure | 0,02 bis 0,25 Gew.-% | 0,005 bis 5 Gew.-% | 7,0 - 6,5 |
| 37 | Äpfelsäure | 0,0001 bis 5 Gew.-% | 0,005 bis 5 Gew.-% | 6,5 - 6,0 |
| 38 | Äpfelsäure | 0,001 bis 2,5 Gew.-% | 0,005 bis 5 Gew.-% | 6,5 - 6,0 |
| 39 | Äpfelsäure | 0,01 bis 1 Gew.-% | 0,005 bis 5 Gew.-% | 6,5 - 6,0 |
| 40 | Äpfelsäure | 0,02 bis 0,25 Gew.-% | 0,005 bis 5 Gew.-% | 6,5 - 6,0 |
| 41 | Äpfelsäure | 0,0001 bis 5 Gew.-% | 0,005 bis 5 Gew.-% | 6,0 - 5,5 |
| 42 | Äpfelsäure | 0,001 bis 2,5 Gew.-% | 0,005 bis 5 Gew.-% | 6,0 - 5,5 |
| 43 | Äpfelsäure | 0,01 bis 1 Gew.-% | 0,005 bis 5 Gew.-% | 6,0 - 5,5 |
| 44 | Äpfelsäure | 0,02 bis 0,25 Gew.-% | 0,005 bis 5 Gew.-% | 6,0 - 5,5 |
| 45 | Äpfelsäure | 0,0001 bis 5 Gew.-% | 0,005 bis 5 Gew.-% | 5,5 - 5,0 |
| 46 | Äpfelsäure | 0,001 bis 2,5 Gew.-% | 0,005 bis 5 Gew.-% | 5,5 - 5,0 |
| 47 | Äpfelsäure | 0,01 bis 1 Gew.-% | 0,005 bis 5 Gew.-% | 5,5 - 5,0 |

(fortgesetzt)

| Nr. | a) anorganische / organische Säure | b) direktziehender Farbstoff | c) Verbindung(en) der Formel (I) | pH-Wert des Mittels |
|---|---|---|---|---|
| 48 | Äpfelsäure | 0,02 bis 0,25 Gew.-% | 0,005 bis 5 Gew.-% | 5,5 - 5,0 |
| 49 | Milchsäure | 0,0001 bis 5 Gew.-% | 0,005 bis 5 Gew.-% | 7,0 - 6,5 |
| 50 | Milchsäure | 0,001 bis 2,5 Gew.-% | 0,005 bis 5 Gew.-% | 7,0 - 6,5 |
| 51 | Milchsäure | 0,01 bis 1 Gew.-% | 0,005 bis 5 Gew.-% | 7,0 - 6,5 |
| 52 | Milchsäure | 0,02 bis 0,25 Gew.-% | 0,005 bis 5 Gew.-% | 7,0 - 6,5 |
| 53 | Milchsäure | 0,0001 bis 5 Gew.-% | 0,005 bis 5 Gew.-% | 6,5 - 6,0 |
| 54 | Milchsäure | 0,001 bis 2,5 Gew.-% | 0,005 bis 5 Gew.-% | 6,5 - 6,0 |
| 55 | Milchsäure | 0,01 bis 1 Gew.-% | 0,005 bis 5 Gew.-% | 6,5 - 6,0 |
| 56 | Milchsäure | 0,02 bis 0,25 Gew.-% | 0,005 bis 5 Gew.-% | 6,5 - 6,0 |
| 57 | Milchsäure | 0,0001 bis 5 Gew.-% | 0,005 bis 5 Gew.-% | 6,0 - 5,5 |
| 58 | Milchsäure | 0,001 bis 2,5 Gew.-% | 0,005 bis 5 Gew.-% | 6,0 - 5,5 |
| 59 | Milchsäure | 0,01 bis 1 Gew.-% | 0,005 bis 5 Gew.-% | 6,0 - 5,5 |
| 60 | Milchsäure | 0,02 bis 0,25 Gew.-% | 0,005 bis 5 Gew.-% | 6,0 - 5,5 |
| 61 | Milchsäure | 0,0001 bis 5 Gew.-% | 0,005 bis 5 Gew.-% | 5,5 - 5,0 |
| 62 | Milchsäure | 0,001 bis 2,5 Gew.-% | 0,005 bis 5 Gew.-% | 5,5 - 5,0 |
| 63 | Milchsäure | 0,01 bis 1 Gew.-% | 0,005 bis 5 Gew.-% | 5,5 - 5,0 |
| 64 | Milchsäure | 0,02 bis 0,25 Gew.-% | 0,005 bis 5 Gew.-% | 5,5 - 5,0 |
| 65 | 1-Hydroxyethan-1,1-diphosphonsäure | 0,0001 bis 5 Gew.-%, | 0,005 bis 5 Gew.-% | 7,0 - 6,5 |
| 66 | 1-Hydroxyethan-1,1-diphosphonsäure | 0,001 bis 2,5 Gew.-%, | 0,005 bis 5 Gew.-% | 7,0 - 6,5 |
| 67 | 1-Hydroxyethan-1,1-diphosphonsäure | 0,01 bis 1 Gew.-% | 0,005 bis 5 Gew.-% | 7,0 - 6,5 |
| 68 | 1-Hydroxyethan-1,1-diphosphonsäure | 0,02 bis 0,25 Gew.-% | 0,005 bis 5 Gew.-% | 7,0 - 6,5 |
| 69 | 1-Hydroxyethan-1,1-diphosphonsäure | 0,0001 bis 5 Gew.-%, | 0,005 bis 5 Gew.-% | 6,5 - 6,0 |
| 70 | 1-Hydroxyethan-1,1-diphosphonsäure | 0,001 bis 2,5 Gew.-%, | 0,005 bis 5 Gew.-% | 6,5 - 6,0 |
| 71 | 1-Hydroxyethan-1,1-diphosphonsäure | 0,01 bis 1 Gew.-% | 0,005 bis 5 Gew.-% | 6,5 - 6,0 |
| 72 | 1-Hydroxyethan-1,1-diphosphonsäure | 0,02 bis 0,25 Gew.-% | 0,005 bis 5 Gew.-% | 6,5 - 6,0 |
| 73 | 1-Hydroxyethan-1,1-diphosphonsäure | 0,0001 bis 5 Gew.-%, | 0,005 bis 5 Gew.-% | 6,0 - 5,5 |
| 74 | 1-Hydroxyethan-1,1-diphosphonsäure | 0,001 bis 2,5 Gew.-%, | 0,005 bis 5 Gew.-% | 6,0 - 5,5 |
| 75 | 1-Hydroxyethan-1,1-diphosphonsäure | 0,01 bis 1 Gew.-% | 0,005 bis 5 Gew.-% | 6,0 - 5,5 |
| 76 | 1-Hydroxyethan-1,1-diphosphonsäure | 0,02 bis 0,25 Gew.-% | 0,005 bis 5 Gew.-% | 6,0 - 5,5 |

(fortgesetzt)

| Nr. | a) anorganische / organische Säure | b) direktziehender Farbstoff | c) Verbindung(en) der Formel (I) | pH-Wert des Mittels |
|---|---|---|---|---|
| 77 | 1-Hydroxyethan-1,1-diphosphonsäure | 0,0001 bis 5 Gew.-%, | 0,005 bis 5 Gew.-% | 5,5 - 5,0 |
| 78 | 1-Hydroxyethan-1,1-diphosphonsäure | 0,001 bis 2,5 Gew.-%, | 0,005 bis 5 Gew.-% | 5,5 - 5,0 |
| 79 | 1-Hydroxyethan-1,1-diphosphonsäure | 0,01 bis 1 Gew.-% | 0,005 bis 5 Gew.-% | 5,5 - 5,0 |
| 80 | 1-Hydroxyethan-1,1-diphosphonsäure | 0,02 bis 0,25 Gew.-% | 0,005 bis 5 Gew.-% | 5,5 - 5,0 |
| 81 | Zitronensäure | 0,0001 bis 5 Gew.-% | Bis-Cetearyl Amodimethicon | 7,0 - 6,5 |
| 82 | Zitronensäure | 0,001 bis 2,5 Gew.-% | Bis-Cetearyl Amodimethicon | 7,0 - 6,5 |
| 83 | Zitronensäure | 0,01 bis 1 Gew.-% | Bis-Cetearyl Amodimethicon | 7,0 - 6,5 |
| 84 | Zitronensäure | 0,02 bis 0,25 Gew.-% | Bis-Cetearyl Amodimethicon | 7,0 - 6,5 |
| 85 | Zitronensäure | 0,0001 bis 5 Gew.-% | Bis-Cetearyl Amodimethicon | 6,5 - 6,0 |
| 86 | Zitronensäure | 0,001 bis 2,5 Gew.-% | Bis-Cetearyl Amodimethicon | 6,5 - 6,0 |
| 87 | Zitronensäure | 0,01 bis 1 Gew.-% | Bis-Cetearyl Amodimethicon | 6,5 - 6,0 |
| 88 | Zitronensäure | 0,02 bis 0,25 Gew.-% | Bis-Cetearyl Amodimethicon | 6,5 - 6,0 |
| 89 | Zitronensäure | 0,0001 bis 5 Gew.-% | Bis-Cetearyl Amodimethicon | 6,0 - 5,5 |
| 90 | Zitronensäure | 0,001 bis 2,5 Gew.-% | Bis-Cetearyl Amodimethicon | 6,0 - 5,5 |
| 91 | Zitronensäure | 0,01 bis 1 Gew.-% | Bis-Cetearyl Amodimethicon | 6,0 - 5,5 |
| 92 | Zitronensäure | 0,02 bis 0,25 Gew.-% | Bis-Cetearyl Amodimethicon | 6,0 - 5,5 |
| 93 | Zitronensäure | 0,0001 bis 5 Gew.-% | Bis-Cetearyl Amodimethicon | 5,5 - 5,0 |
| 94 | Zitronensäure | 0,001 bis 2,5 Gew.-% | Bis-Cetearyl Amodimethicon | 5,5 - 5,0 |
| 95 | Zitronensäure | 0,01 bis 1 Gew.-% | Bis-Cetearyl Amodimethicon | 5,5 - 5,0 |
| 96 | Zitronensäure | 0,02 bis 0,25 Gew.-% | Bis-Cetearyl Amodimethicon | 5,5 - 5,0 |
| 97 | Weinsäure | 0,0001 bis 5 Gew.-% | Bis-Cetearyl Amodimethicon | 7,0 - 6,5 |
| 98 | Weinsäure | 0,001 bis 2,5 Gew.-% | Bis-Cetearyl Amodimethicon | 7,0 - 6,5 |

(fortgesetzt)

| Nr. | a) anorganische / organische Säure | b) direktziehender Farbstoff | c) Verbindung(en) der Formel (I) | pH-Wert des Mittels |
|---|---|---|---|---|
| 99 | Weinsäure | 0,01 bis 1 Gew.-% | Bis-Cetearyl Amodimethicon | 7,0 - 6,5 |
| 100 | Weinsäure | 0,02 bis 0,25 Gew.-% | Bis-Cetearyl Amodimethicon | 7,0 - 6,5 |
| 101 | Weinsäure | 0,0001 bis 5 Gew.-% | Bis-Cetearyl Amodimethicon | 6,5 - 6,0 |
| 102 | Weinsäure | 0,001 bis 2,5 Gew.-% | Bis-Cetearyl Amodimethicon | 6,5 - 6,0 |
| 103 | Weinsäure | 0,01 bis 1 Gew.-% | Bis-Cetearyl Amodimethicon | 6,5 - 6,0 |
| 104 | Weinsäure | 0,02 bis 0,25 Gew.-% | Bis-Cetearyl Amodimethicon | 6,5 - 6,0 |
| 105 | Weinsäure | 0,0001 bis 5 Gew.-% | Bis-Cetearyl Amodimethicon | 6,0 - 5,5 |
| 106 | Weinsäure | 0,001 bis 2,5 Gew.-% | Bis-Cetearyl Amodimethicon | 6,0 - 5,5 |
| 107 | Weinsäure | 0,01 bis 1 Gew.-% | Bis-Cetearyl Amodimethicon | 6,0 - 5,5 |
| 108 | Weinsäure | 0,02 bis 0,25 Gew.-% | Bis-Cetearyl Amodimethicon | 6,0 - 5,5 |
| 109 | Weinsäure | 0,0001 bis 5 Gew.-% | Bis-Cetearyl Amodimethicon | 5,5 - 5,0 |
| 110 | Weinsäure | 0,001 bis 2,5 Gew.-% | Bis-Cetearyl Amodimethicon | 5,5 - 5,0 |
| 111 | Weinsäure | 0,01 bis 1 Gew.-% | Bis-Cetearyl Amodimethicon | 5,5 - 5,0 |
| 112 | Weinsäure | 0,02 bis 0,25 Gew.-% | Bis-Cetearyl Amodimethicon | 5,5 - 5,0 |
| 113 | Äpfelsäure | 0,0001 bis 5 Gew.-% | Bis-Cetearyl Amodimethicon | 7,0 - 6,5 |
| 114 | Äpfelsäure | 0,001 bis 2,5 Gew.-% | Bis-Cetearyl Amodimethicon | 7,0 - 6,5 |
| 115 | Äpfelsäure | 0,01 bis 1 Gew.-% | Bis-Cetearyl Amodimethicon | 7,0 - 6,5 |
| 116 | Äpfelsäure | 0,02 bis 0,25 Gew.-% | Bis-Cetearyl Amodimethicon | 7,0 - 6,5 |
| 117 | Äpfelsäure | 0,0001 bis 5 Gew.-% | Bis-Cetearyl Amodimethicon | 6,5 - 6,0 |
| 118 | Äpfelsäure | 0,001 bis 2,5 Gew.-% | Bis-Cetearyl Amodimethicon | 6,5 - 6,0 |
| 119 | Äpfelsäure | 0,01 bis 1 Gew.-% | Bis-Cetearyl Amodimethicon | 6,5 - 6,0 |
| 120 | Äpfelsäure | 0,02 bis 0,25 Gew.-% | Bis-Cetearyl Amodimethicon | 6,5 - 6,0 |

(fortgesetzt)

| Nr. | a) anorganische / organische Säure | b) direktziehender Farbstoff | c) Verbindung(en) der Formel (I) | pH-Wert des Mittels |
|---|---|---|---|---|
| 121 | Äpfelsäure | 0,0001 bis 5 Gew.-% | Bis-Cetearyl Amodimethicon | 6,0 - 5,5 |
| 122 | Äpfelsäure | 0,001 bis 2,5 Gew.-% | Bis-Cetearyl Amodimethicon | 6,0 - 5,5 |
| 123 | Äpfelsäure | 0,01 bis 1 Gew.-% | Bis-Cetearyl Amodimethicon | 6,0 - 5,5 |
| 124 | Äpfelsäure | 0,02 bis 0,25 Gew.-% | Bis-Cetearyl Amodimethicon | 6,0 - 5,5 |
| 125 | Äpfelsäure | 0,0001 bis 5 Gew.-% | Bis-Cetearyl Amodimethicon | 5,5 - 5,0 |
| 126 | Äpfelsäure | 0,001 bis 2,5 Gew.-% | Bis-Cetearyl Amodimethicon | 5,5 - 5,0 |
| 127 | Äpfelsäure | 0,01 bis 1 Gew.-% | Bis-Cetearyl Amodimethicon | 5,5 - 5,0 |
| 128 | Äpfelsäure | 0,02 bis 0,25 Gew.-% | Bis-Cetearyl Amodimethicon | 5,5 - 5,0 |
| 129 | Milchsäure | 0,0001 bis 5 Gew.-% | Bis-Cetearyl Amodimethicon | 7,0 - 6,5 |
| 130 | Milchsäure | 0,001 bis 2,5 Gew.-% | Bis-Cetearyl Amodimethicon | 7,0 - 6,5 |
| 131 | Milchsäure | 0,01 bis 1 Gew.-% | Bis-Cetearyl Amodimethicon | 7,0 - 6,5 |
| 132 | Milchsäure | 0,02 bis 0,25 Gew.-% | Bis-Cetearyl Amodimethicon | 7,0 - 6,5 |
| 133 | Milchsäure | 0,0001 bis 5 Gew.-% | Bis-Cetearyl Amodimethicon | 6,5 - 6,0 |
| 134 | Milchsäure | 0,001 bis 2,5 Gew.-% | Bis-Cetearyl Amodimethicon | 6,5 - 6,0 |
| 135 | Milchsäure | 0,01 bis 1 Gew.-% | Bis-Cetearyl Amodimethicon | 6,5 - 6,0 |
| 136 | Milchsäure | 0,02 bis 0,25 Gew.-% | Bis-Cetearyl Amodimethicon | 6,5 - 6,0 |
| 137 | Milchsäure | 0,0001 bis 5 Gew.-% | Bis-Cetearyl Amodimethicon | 6,0 - 5,5 |
| 138 | Milchsäure | 0,001 bis 2,5 Gew.-% | Bis-Cetearyl Amodimethicon | 6,0 - 5,5 |
| 139 | Milchsäure | 0,01 bis 1 Gew.-% | Bis-Cetearyl Amodimethicon | 6,0 - 5,5 |
| 140 | Milchsäure | 0,02 bis 0,25 Gew.-% | Bis-Cetearyl Amodimethicon | 6,0 - 5,5 |
| 141 | Milchsäure | 0,0001 bis 5 Gew.-% | Bis-Cetearyl Amodimethicon | 5,5 - 5,0 |
| 142 | Milchsäure | 0,001 bis 2,5 Gew.-% | Bis-Cetearyl Amodimethicon | 5,5 - 5,0 |

(fortgesetzt)

| Nr. | a) anorganische / organische Säure | b) direktziehender Farbstoff | c) Verbindung(en) der Formel (I) | pH-Wert des Mittels |
|---|---|---|---|---|
| 143 | Milchsäure | 0,01 bis 1 Gew.-% | Bis-Cetearyl Amodimethicon | 5,5 - 5,0 |
| 144 | Milchsäure | 0,02 bis 0,25 Gew.-% | Bis-Cetearyl Amodimethicon | 5,5 - 5,0 |
| 145 | 1-Hydroxyethan-1,1-diphosphonsäure | 0,0001 bis 5 Gew.-%, | Bis-Cetearyl Amodimethicon | 7,0 - 6,5 |
| 146 | 1-Hydroxyethan-1,1-diphosphonsäure | 0,001 bis 2,5 Gew.-%, | Bis-Cetearyl Amodimethicon | 7,0 - 6,5 |
| 147 | 1-Hydroxyethan-1,1-diphosphonsäure | 0,01 bis 1 Gew.-% | Bis-Cetearyl Amodimethicon | 7,0 - 6,5 |
| 148 | 1-Hydroxyethan-1,1-diphosphonsäure | 0,02 bis 0,25 Gew.-% | Bis-Cetearyl Amodimethicon | 7,0 - 6,5 |
| 149 | 1-Hydroxyethan-1,1-diphosphonsäure | 0,0001 bis 5 Gew.-%, | Bis-Cetearyl Amodimethicon | 6,5 - 6,0 |
| 150 | 1-Hydroxyethan-1,1-diphosphonsäure | 0,001 bis 2,5 Gew.-%, | Bis-Cetearyl Amodimethicon | 6,5 - 6,0 |
| 151 | 1-Hydroxyethan-1,1-diphosphonsäure | 0,01 bis 1 Gew.-% | Bis-Cetearyl Amodimethicon | 6,5 - 6,0 |
| 152 | 1-Hydroxyethan-1,1-diphosphonsäure | 0,02 bis 0,25 Gew.-% | Bis-Cetearyl Amodimethicon | 6,5 - 6,0 |
| 153 | 1-Hydroxyethan-1,1-diphosphonsäure | 0,0001 bis 5 Gew.-%, | Bis-Cetearyl Amodimethicon | 6,0 - 5,5 |
| 154 | 1-Hydroxyethan-1,1-diphosphonsäure | 0,001 bis 2,5 Gew.-%, | Bis-Cetearyl Amodimethicon | 6,0 - 5,5 |
| 155 | 1-Hydroxyethan-1,1-diphosphonsäure | 0,01 bis 1 Gew.-% | Bis-Cetearyl Amodimethicon | 6,0 - 5,5 |
| 156 | 1-Hydroxyethan-1,1-diphosphonsäure | 0,02 bis 0,25 Gew.-% | Bis-Cetearyl Amodimethicon | 6,0 - 5,5 |
| 157 | 1-Hydroxyethan-1,1-diphosphonsäure | 0,0001 bis 5 Gew.-%, | Bis-Cetearyl Amodimethicon | 5,5 - 5,0 |
| 158 | 1-Hydroxyethan-1,1-diphosphonsäure | 0,001 bis 2,5 Gew.-%, | Bis-Cetearyl Amodimethicon | 5,5 - 5,0 |
| 159 | 1-Hydroxyethan-1,1-diphosphonsäure | 0,01 bis 1 Gew.-% | Bis-Cetearyl Amodimethicon | 5,5 - 5,0 |
| 160 | 1-Hydroxyethan-1,1-diphosphonsäure | 0,02 bis 0,25 Gew.-% | Bis-Cetearyl Amodimethicon | 5,5 - 5,0 |

Es hat sich herausgestellt, dass der Zusatz eines organischen Lösungsmittels das Glanzergebnis weiter verbessert. Als Lösungsmittel können 2-Butoxyethanol, Propylenglykolmonomethylether, Diethylengkykolmonomethylether, Diethylenglykolmonoethylether, Ethylenglykolmonobutylether, Ethylenglykolmonoethylether, Ethanol, Isopropanol, n-Propanol, n-Butanol, 1,3-Propandiol, Glycerin, 1-Methoxy-2-propanol, 1-Ethoxy-2-propandiol, 1,4-Butandiol, 1,3-Butandiol, 1,2-Hexandiol, Benzylalkohol, Phenoxyethanol, 2-Phenylethylalkohol, Methoxybutanol, n-Butylenglykol, Ethylencarbonat und Propylencarbonat eingesetzt werden.

Bevorzugte Lösungsmittel sind Lösungsmittel, welche mindestens eine Hydroxygruppe enthalten, beispielsweise Ethanol, Isopropanol, n-Propanol, 1,3-Propandiol, Glycerin, 1-Methoxy-2-propanol, 1-Ethoxy-2-propandiol, 1,4-Butandiol, 1,3-Butandiol, 1,2-Hexandiol, Benzylalkohol, Phenoxyethanol, 2-Phenylethylalkohol, Methoxybutanol und n-Butylenglykol.

Besonders bevorzugt sind Lösungsmittel, welche mindestens zwei Hydroxygruppen enthalten, beispielsweise , 1,3-Propandiol, Glycerin, 1-Ethoxy-2-propandiol, 1,4-Butandiol, 1,3-Butandiol, 1,2-Hexandiol, und n-Butylenglykol.

Die Lösungsmittel können in dem erfindungsgemäßen Mittel in einer Gesamtmenge von 0,1 bis 15 Gew.-%, bevorzugt 1 bis 10 Gew.-%, weiter bevorzugt 1,5 bis 8 Gew.-% und insbesondere bevorzugt 2 bis 6 Gew.-%, bezogen auf sein Gesamtgewicht, enthalten sein.

In einer weiteren Ausführungsform ist ein erfindungsgemäßes Mittel dadurch gekennzeichnet, dass es ein oder mehrere Lösungsmittel aus der Gruppe 2-Butoxyethanol, Propylenglykolmonomethylether, Diethylengkykolmonomethylether, Diethylenglykolmonoethylether, Ethylenglykolmonobutylether, Ethylenglykolmonoethylether, Ethanol, Isopropanol, n-Propanol, n-Butanol, 1,3-Propandiol, Glycerin, 1-Methoxy-2-propanol, 1-Ethoxy-2-propandiol, 1,4-Butandiol, 1,3-Butandiol, 1,2-Hexandiol, Benzylalkohol, Phenoxyethanol, 2-Phenylethylalkohol, Methoxybutanol, n-Butylenglykol, Ethylencarbonat und Propylencarbonat in einer Gesamtmenge von 0,1 bis 15 Gew.-%, bevorzugt 1 bis 10 Gew.-%, weiter bevorzugt 1,5 bis 8 Gew.-% und insbesondere bevorzugt 2 bis 6 Gew.-%, bezogen auf das Gesamtgewicht des Mittels, enthält.

Des Weiteren konnte bei den zu dieser Erfindung führenden Arbeiten gefunden werden, dass auch der Zusatz eines oder mehrerer Polyethylenglykole und/oder Polypropylenglykole das Glanzresultat noch weiter zu steigern vermag. In einer weiteren bevorzugten Ausführungsform enthalten die erfindungsgemäßen Mittel daher zusätzlich mindestens ein Polyethylenglykol und/oder Poylpropylengkol in einer Gesamtmenge von 0,1 bis 15 Gew.-%, bevorzugt 1 bis 10 Gew.-%, weiter bevorzugt 1,5 bis 8 Gew.-% und insbesondere bevorzugt 2 bis 6 Gew.-%, bezogen auf das Gesamtgewicht des Mittels.

In einer weiteren bevorzugten Ausführungsform ist ein erfindungsgemäßes Mittel dadurch gekennzeichnet, dass es eine oder mehrere Verbindungen der Formel (II)

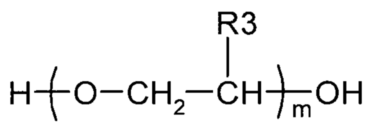

(II)

worin

R3 für ein Wasserstoffatom oder eine Methylgruppe steht und
m für eine Zahl von 2 bis 1000 steht,
in einem Gesamtgewicht von 0,1 bis 15 Gew.-%, bevorzugt 1 bis 10 Gew.-%, weiter bevorzugt 1,5 bis 8 Gew.-% und insbesondere bevorzugt 2 bis 6 Gew.-%, bezogen auf das Gesamtgewicht des Mittels, enthält.

Der Rest R3 kann hierbei für ein Wasserstoffatom oder eine Methylgruppe stehen. Bevorzugt steht R3 für ein Wasserstoffatom.

m steht für eine Zahl von 2 bis 100. Bevorzugt steht m für eine Zahl von 10 bis 600, besonders bevorzugt für eine Zahl von 40 bis 500.

Auch wenn die erfindungsgemäßen Mittel bevorzugt auf einen neutralen bis sauren pH-Wert eingestellt sind, so können sie dennoch prinzipiell alkalisch reagierende Verbindungen wie Ammoniak oder aliphatische Alkanolamine enthalten, die im Fall der vorliegenden Erfindung als Penetrationsmittel wirken können und so eine Penetration der aktiven Spezies in das Haar hinein ermöglichen. Zur Erzielung der gewünschten pH-Werte werden die Mengen der erfindungsgemäß eingesetzten Säuren dann entsprechend erhöht.

In einer weiteren Ausführungsform ist das erfindungsgemäße Mittel daher dadurch gekennzeichnet, dass es, bezogen auf das Gesamtgewicht des Mittels, 0,01 bis 15 Gew.-%, vorzugsweise 0,1 bis 10 Gew.-%, besonders bevorzugt 1 bis 8 Gew.-% und insbesondere bevorzugt 3 bis 7 Gew.-% einer Verbindung ausgewählt aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol,

1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol enthält.

Werden die erfindungsgemäßen Mittel zum oxidativen Färben und/oder Aufhellen von Haaren eingesetzt, so können sie als weiteren Bestandteil neben der anorganischen/organischen Säuren, dem direktziehenden Farbstoff und der/den Verbindung(en) der Formel (I) mindestens ein Oxidationsfarbstoffvorprodukt vom Entwickler und/oder Kupplertyp enthalten.

Erfindungsgemäß sind solche Mittel bevorzugt, die die Entwickler und/oder Kupplerkomponenten jeweils in

einem Gewichtsanteil von 0,001 bis 10 Gew.-%, bevorzugt, 0,01 bis 8 Gew.-%, besonders bevorzugt 0,1 bis 5 Gew.-% und insbesondere bevorzugt 0,5 bis 3 Gew.-%, bezogen auf das Gesamtgewicht des Mittels, enthalten.

In einer weiteren bevorzugten Ausführungsform ist das erfindungsgemäße Mittel daher dadurch gekennzeichnet, dass es ein Oxidationsfarbstoffvorprodukt vom Entwickler und/oder Kupplertyp in einem Gewichtsanteil von 0,001 bis 10 Gew.-%, bevorzugt 0,01 bis 8 Gew.-%, besonders bevorzugt 0,1 bis 5 Gew.-% und insbesondere bevorzugt 0,5 bis 3 Gew.-%, bezogen auf das Gesamtgewicht des Mittels, enthält.

In einer bevorzugten Ausführungsform des ersten Erfindungsgegenstands enthält das Mittel als Oxidationsfarbstoffvorprodukt mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 3 : 1 bis 1 : 3, insbesondere 2 : 1 bis 1 : 1, enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylendiamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methylphenyl)-N-[3-(1 H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden ausgewählt N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan und Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol sowie deren physiologisch verträglichen Salze.

Ferner kann die Entwicklerkomponente ausgewählt sein aus o-Aminophenol und seinen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind die Verbindungen 2,4,5,6-Tetraaminopyrimidin, 4-Hydroxy-2,5,6-triaminopyrimidin und deren physiologisch verträglichen Salze. Bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt. Bevorzugte Oxidationsfarbstoffvorprodukte vom Entwicklertyp sind ausgewählt aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen. Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Eine weitere bevorzugte Ausführungsform der vorliegenden Erfindung ist dadurch gekennzeichnet, dass das erfindungsgemäße Mittel als Oxidationsfarbstoffvorprodukt neben mindestens einer Entwicklerkomponente weiterhin zusätzlich mindestens eine Kupplerkomponente enthält. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)-ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methyl-amino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie eines deren physiologisch verträglichen Salze.

Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden: p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin;p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)-benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1 H-imidazol-1-yl)propyl]amin / 2-Methylresorcin; N-(4-Amino-3-methylphenyl)-N-[3-(1 H-imidazol-1-yl)propyl]amin / 5-Amino-2-methylphenol; N-(4-Amino-3-methylphenyl)-N-[3-(1 H-imidazol-1-yl)propyl]amin / 3-Aminophenol; N-(4-Amino-3-methylphenyl)-N-[3-(1H-imidazol-1-yl)propyl]-amin / 2-(2,4-Diaminophenoxy)ethanol; N-(4-Amino-3-methylphenyl)-N-[3-(1 H-imidazol-1-yl)propyl]amin / 1,3-Bis(2,4-diaminophenoxy)propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1 H-imidazol-1-yl)propyl]amin / 2-Amino-3-hydroxypyridin; N-(4-Amino-3-methylphenyl)-N-[3-(1 H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)-benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

In einer bevorzugten Ausführungsform sind die erfindungsgemäßen Mittel jedoch frei von Oxidationsfarbstoffvorprodukten.

In einer weiteren Ausführungsform der vorliegenden Erfindung enthalten die erfindungsgemäßen Mittel weiterhin zusätzlich mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Besonders bevorzugte Derivate des Indolins sind das 5,6-Dihydroxyindolin und das 2,3-Dioxoindolin (Isatin) und deren physiologisch verträglichen Salze. Ein besonders bevorzugtes Derivat des Indols ist das 5,6-Dihydroxyindol und dessen physiologisch verträglichen Salze. Die erfindungsgemäßen Mittel enthalten die Indol- oder Indolin-Derivate vorzugsweise in einem Gewichtsanteil von 0,05 bis 10 Gew.-%, bevorzugt 0,2 bis 5 Gew.-%, jeweils bezogen auf ihr Gesamtgewicht.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Wenn das erfindungsgemäße Mittel als oxidatives Färbemittel angewendet werden soll, so wird es bevorzugt unmittelbar vor der Anwendung durch Vermischen mindestens zweier Zubereitungen hergestellt, wobei die mindestens zwei Zubereitungen in mindestens zwei getrennt konfektionierten Containern bereitgestellt werden und wobei ein Container ein Färbe- und/oder Aufhellmittel (A) gemäß des ersten Erfindungsgegenstandes enthält, und ein weiterer Container eine Oxidationsmittelzubereitung (B), enthaltend mindestens ein Oxidationsmittel, enthält.

Als Oxidationsmittel können Wasserstoffperoxid sowie seine festen Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Insbesondere bevorzugt wird als Oxidationsmittel Wasserstoffperoxid eingesetzt. Bevorzugt beträgt der Gewichtsanteil an Wasserstoffperoxid im anwendungsbereiten Mittel 0,5 bis 12 Gew.-%, vorzugsweise 2 bis 10 Gew.-% und insbesondere bevorzugt 3 bis 6 Gew.-% (berechnet als 100 %-iges $H_2O_2$), jeweils bezogen auf das Gesamtgewicht des Mittels.

Ein weitere bevorzugte Ausführungsform des ersten Erfindungsgegenstands ist daher ein anwendungsbereites Mittel, welches dadurch gekennzeichnet ist, dass es zusätzlich als Oxidationsmittel Wasserstoffperoxid und/oder eines seiner festen Anlagerungsprodukte an organische oder anorganische Verbindungen in einem Gewichtsanteil von 0,5 bis 12 Gew.-%, vorzugsweise 2 bis 10 Gew.-% und insbesondere bevorzugt 3 bis 6 Gew.-% (berechnet als 100 %-iges $H_2O_2$), bezogen auf das Gesamtgewicht des Mittels, enthält.

Das anwendungsbereite Mittel kann in einer besonderen Ausführungsform der Erfindung erst kurz vor der Anwendung aus einer Farbveränderungszubereitung und einer Oxidationsmittelzubereitung hergestellt werden.

Solche Oxidationsmittelzubereitungen sind vorzugsweise wässrige Oxidationsmittelzubereitungen. Dabei sind bevorzugte Zubereitungen dadurch gekennzeichnet, dass die Oxidationsmittelzubereitung, bezogen auf ihr Gewicht, 40 bis 95 Gew.-%, vorzugsweise 50 bis 90 Gew.-%, besonders bevorzugt 55 bis 80 Gew.-% Wasser enthält.

Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Weiterhin hat es sich als vorteilhaft erweisen, wenn die Mittel, insbesondere die Oxidationsmittelzubereitungen, mindestens einen Stabilisator oder Komplexbildner enthalten. Im Rahmen der vorliegenden Erfindung bevorzugte Chelatkomplexbildner sind beispielsweise von der Säure a) verschiedene Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Aufhellmitteln eingesetzt, woraus eine Steigerung des Aufhellvermögens der Mittel resultiert. Bevorzugte Aufhellmittel sind Bleichkraftverstärker. Hierzu zählen Peroxo-Salze, Siliciumdioxid-Verbindungen sowie insbesondere kationisierte Heterocyclen.

Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Der Einsatz von Persulfatsalzen bzw. Peroxodisulfatsalzen erfolgt in der Regel in Form eines gegebenenfalls entstaubten Pulvers.

Die Aufhellmittel sind in dem Mittel bevorzugt in einem Gewichtsanteil von 0,1 bis 25 Gew.-%, insbesondere in einer Menge von 0,5 bis 15 Gew.-%, bezogen auf das Gesamtgewicht des anwendungsbereiten Mittels, enthalten.

Bezüglich weiterer fakultativer Wirk- und Inhaltsstoffe der Oxidationsmittelzubereitung gilt mutatis mutandis das zu den erfindungsgemäßen Mitteln Gesagte.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Seifen, Alkylsulfate, Alkylethersulfate und Ethercarbonsäuren mit 8 bis 22 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder SulfatGruppe tragen. Besonders geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine COOH- oder $SO_3H$-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet. Weiterhin hat es sich als vorteilhaft erwiesen, wenn die erfindungsgemäßen Färbe- oder Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe.

Als nichtionische Tenside eignen bevorzugt sich Alkylpolyglykoside, insbesondere $C_8$-$C_{22}$-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Erfindungsgemäß besonders bevorzugt sind gesättigte oder ungesättigte $C_{10}$-$C_{22}$ Fettalkohole mit jeweils 2 bis 12 Mol Ethylenoxid pro Mol Fettalkohol (z.B. Laureth-2 oder Ceteareth-20). Zubereitungen mit hervorragenden Eigenschaften werden ebenfalls erhalten, wenn sie als nichtionische Tenside Fettsäureester von ethoxyliertem Glycerin enthalten. Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt. Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt. Insbesondere bevorzugt wird in den erfindungsgemäßen Mitteln mindestens ein amphoteres bzw. zwitterionisches Tensid eingesetzt.

In einer weiteren Ausführungsform des ersten Erfindungsgegenstands ist ein erfindungsgemäßes Mittel dadurch gekennzeichnet, dass es zusätzlich mindestens ein amphoteres und/oder zwitterionisches Tensid enthält.

Entsprechend sind die Mittel besonders bevorzugt, welche

a) mindestens eine anorganische und/oder organische Säure
b) mindestens einen direktziehenden Farbstoff und
c) mindestens eine Verbindung der weiter oben beschriebenen Formel (I) und
d) mindestens ein amphoteres und/oder zwitterionisches Tensid enthalten.

Ferner können die erfindungsgemäßen Mittel weitere Wirk-, Hilfs- und Zusatzstoffe enthalten, wie beispielsweise Assoziativpolymere mit Fettalkylkette, kationische Polymere, nichtionische Polymere (Vinylpyrrolidinon/Vinylacrylat-Copolymere, Polyvinylpyrrolidinon, Vinylpyrrolidinon/Vinylacetat-Copolymere, Polyethylenglykole und Polysiloxane); zwitterionische und amphotere Polymere (Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere); anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); Strukturanden (Glusose, Maleinsäure und Milchsäure), haarkon-ditionierende Verbindungen (Phospholipide, Sojalecithin, Ei-Lecitin, Kephaline sowie Silikonöle); zusätzliche Proteinhydrolysate pflanzlicher oder tierischer Herkunft (Elastin-, Collagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine); Wirkstoffe (Panthenol, Panthothensäure, Pantolacton, Allantoin, Pyrrolidinoncarbonsäuren und deren Salze, Bisabolol, Carnitin, Coffein, Theobromin und Taurin); Vitamine, Provitamine und Vitaminvorstufen, insbesondere solche der Gruppen A, $B_3$, $B_5$, $B_6$, C, E, F und H; Pflanzenextrakte (aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Moringa, Hauhechel, Huflattich, Eibisch, Meristem, Ginseng und Ingwer); pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl, Teebaumöl); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol / Acrylamid-Copolymere); Perlglanzmittel (Ethylenglykolmonostearat sowie PEG-3-distearat); Treibmittel wie Propan-Butan-Gemische, $N_2O$, Dimethylether, $CO_2$ und Luft; Antioxidantien.

Mittel enthaltend mindestens ein kationisches Polymer sind im Hinblick auf die Lösung der erfindungsgemäßen Aufgabenstellung ebenfalls bevorzugt. Entsprechend sind die Mittel besonders bevorzugt, welche

a) mindestens eine anorganische und/oder organische Säure
b) mindestens einen direktziehenden Farbstoff und
c) mindestens eine Verbindung der weiter oben beschriebenen Formel (I) und
d) mindestens ein kationisches Polymer enthalten.

In einer weiteren Ausführungsform des ersten Erfindungsgegenstands ist ein erfindungsgemäßes Mittel dadurch gekennzeichnet, dass es mindestens ein kationisches Polymer aus der Gruppe Polyerquaternium-4, Polyquaternium-6, Polyquaternium-7, Polyquaternium-8, Polyquaternium-10, Polyquaternium-11, Polyquaternium-22, Polyquaternium-24, Polyquaternium-32, Polyquaternium-37, Polyquatenrium-39, Polyqaternium-44, Polyquaternium-46, Polyquaternium-53, Polyquaternium-55, Polyquaternium-64, Polyquatenrium-68, Polyquaternium-69, Polyquaternium-87 und Polyquaternium-91 enthält.

Das bzw. die kationischen Polymere können in einer Gesamtmenge von 0,01 bis 25 Gew.-%, bevorzugt von 0,1 bis 10 Gew.-%, insbesondere von 0,5 bis 5 Gew.-% in dem erfindungsgemäßen Mittel enthalten sein.

In einer ganz besonders bevorzugten Ausführungsform enthält das erfindungsgemäße Mittel zusätzlich 0,5 bis 5 Gew.-% Polyquatenium-6. In einer weiteren bevorzugten Ausführungsform ist das erfindungsgemäße Mittel dadurch gekennzeichnet, dass es 0,5 bis 5 Gew.-% Polyquaternium-6 enthält.

Das erfindungsgemäße Mittel kann weiterhin zusätzlich mindestens einen Wirk-, Hilfs- und Zusatzstoff aus der Gruppe enthalten, die gebildet wird aus

a. Strukturanten ausgewählt aus Glucose, Maleinsäure und Milchsäure,
b. haarkonditionierenden Verbindungen ausgewählt aus Phospholipiden, Sojalecithin, Ei-Lecitin, Kephalinen und Silikonölen,
c. Proteinhydrolysaten ausgewählt aus Elastin-, Kollagen-, Keratin-, Milcheiweiß-, Sojaprotein- und Weizenproteinhydrolysaten, deren Kondensationsprodukte mit Fettsäuren sowie quaternisierten Proteinhydrolysaten,
d. Wirkstoffen ausgewählt aus Panthenol, Pantothensäure, Pantolacton, Allantoin, Pyrrolidoncarbonsäuren und deren Salzen, Bisabolol, Carnitin, Coffein, Theophyllin, Theobromin und Taurin,
e. Vitaminen, Provitaminen und Vitaminvorstufen und
f. Pflanzenextrakten.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in einem Gewichtsanteil von 0,01 bis 25 Gew.-%, insbesondere 0,05 bis 15 Gew.-%, bezogen auf die Gesamtmenge des anwendungsbereiten Mittels.

Bevorzugt handelt es sich bei dem erfindungsgemäßen Mittel um ein Einkomponentenmittel. Die erfindungsgemäßen Mittel können jedoch auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Im Falle eines oxidativen Färbemittels, bei dem die Oxidationsfarbstoffvorprodukte zunächst getrennt von einer Oxidationsmittelzubereitung, enthaltend bevorzugt Wasserstoffperoxid, vorliegen, ist dieses Vorgehen besonders bevorzugt.

Ein weiterer Erfindungsgegenstand ist daher eine Mehrkomponenten- Verpackungseinheit ("Kit-ofparts") zum Färben und/oder Aufhellen keratinischer Fasern, wobei die Mehrkomponenten Verpackungseinheit einen ersten separat verpackten Container (A) enthält, der mindestens ein Mittel gemäß des ersten Erfindungsgegenstands enthält, und mindestens einen zweiten separat verpackten Container (B) enthält, der mindestens ein Mittel enthaltend Wasserstoffperoxid, enthält.

Die Mittel des ersten Erfindungsgegenstandes können zum Färben und/oder Nuancieren und, sofern sie zusätzlich ein Oxidationsmittel enthalten, auch zum Aufhellen von Haaren verwendet werden. Gleichzeitig mit der Färbung bzw. Nuancierung erzeugen die erfindungsgemäßen Mittel einen starken, lang anhaltenden Haarglanz.

Ein weiterer Gegenstand der vorliegenden Erfindung ist daher die kosmetische, nicht-therapeutische Verwendung eines Mittels des ersten Erfindungsgegenstandes zur Erzeugung von lang anhaltendem Glanz auf keratinischen Fasern.

Die Mittel des ersten Erfindungsgegenstands können in Verfahren zum Färben, Nuancieren und/oder Aufhellen menschlicher Haare genutzt werden. Werden die Mittel als Nuanciermittel eingesetzt, so kann die Nuancierung direkt im Anschluss, d.h. einige Stunden oder maximal einen Tag nach einer bereits durchgeführten Färbung vorgenommen werden. Es ist jedoch ebenfalls denkbar, dass zwischen der Färbung und der mit dem erfindungsgemäßen Mittel vorgenommenen Nuancierung ein Zeitraum von einer bis mehreren Wochen, bevorzugt von 10 bis 14 Tagen, liegt.

Für die Färbung bzw. Nuancierung wird ein Mittel des ersten Erfindungsgegenstands auf das Haar aufgetragen und für einen Zeitraum von 30 Sekunden bis 45 Minuten, bevorzugt 1 bis 30 Minuten, besonders bevorzugt von 2 bis 15 Minuten, im Haar belassen. Anschließend wird das Haar mit Wasser und/oder einem handelsüblichen Shampoo gespült. Gewünschtenfalls kann das Haar anschließend mit einem weiteren Mittel behandelt werden und danach erneut mit Wasser und/oder einem handelsüblichen Shampoo gespült werden.

Ein weiterer Gegenstand der vorliegenden Erfindung ist daher ein Verfahren zur Erzeugung von lang anhaltendem Glanz auf keratinischen Fasern, insbesondere menschlichen Haaren, bei welchem ein Mittel des ersten Erfindungsgegenstandes auf die keratinischen Fasern aufgetragen, dort für 30 Sekunden bis 45 Minuten belassen und anschließend mit Wasser oder einem tensidhaltigen Mittel wieder ausgespült wird.

Die Auftragungs- und die Einwirktemperatur der Färbe- und/oder Aufhellzubereitung beträgt Raumtemperatur bis 45°C. Gegebenenfalls kann die Wirkung der Färbe- und/oder Aufhellzubereitung durch externe Wärmezufuhr, wie beispielsweise mittels einer Wärmehaube, verstärkt werden. Nach Beendigung der Einwirkdauer wird das verbliebene Färbe- und/oder Aufhellmittel aus den keratinischen Fasern mit Hilfe einer Reinigungszubereitung oder Wasser ausgewaschen. Gegebenenfalls wird der Vorgang mit einem weiteren Mittel wiederholt. Nach dem Auswaschen werden die keratinischen Fasern gegebenenfalls mit einem Handtuch oder einem Heißluftgebläse getrocknet. Die Auftragung der Färbe- und/oder Aufhellzubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder

Latex zur einmaligen Benutzung (Einweghandschuhe) sowie gegebenenfalls eine Schürze. Es ist aber auch möglich, die Färbe- und/oder Aufhellmittel mit einer Applikationshilfe auf die keratinischen Fasern aufzutragen.

Bezüglich weiterer bevorzugter Ausführungsformen der erfindungsgemäßen Verfahren und Verwendungen gilt mutatis mutandis das zu den erfindungsgemäßen Mitteln Gesagte.

BEISPIELE

Die Mengenangaben verstehen sich, soweit nichts anderes vermerkt ist, jeweils in Gewichtsprozent.

1.1. Formulierungen

Es wurden die nachfolgenden Formulierungen hergestellt. Bei V1 bis V3 handelt es sich um nicht erfindungsgemäße Vergleichsformulierungen, E ist eine erfindungsgemäße Rezeptur.

| Beschreibung | V1 | V2 | V3 | E |
|---|---|---|---|---|
| Lorol $C_{12}$-$C_{18}$ techn. | 4,0 | 4,0 | 4,0 | 4,0 |
| Cocoamidopropylbetain | 4,5 | 4,5 | 4,5 | 4,5 |
| Texapon NSO | 4,0 | 4,0 | 4,0 | 4,0 |
| Dehydol LS 2 Deo N | 0,8 | 0,8 | 0,8 | 0,8 |
| Emulgade 1000 NI | 2,4 | 2,4 | 2,4 | 2,4 |
| Methylparaben | 0,4 | 0,4 | 0,4 | 0,4 |
| Propylparaben | 0,19 | 0,19 | 0,19 | 0,19 |
| Polyethylenglykol | 4,0 | 4,0 | 4,0 | 4,0 |
| 1,3-Butandiol | 1,0 | 1,0 | 1,0 | 1,0 |
| Carbomer | 1,0 | 1,0 | 1,0 | 1,0 |
| Polyquaternium-6 | 1,0 | 1,0 | 1,0 | 1,0 |
| HC Yellow 2 | 0,013 | --- | --- | 0,013 |
| HC Blue 12 | 0,04 | --- | --- | 0,04 |
| 2-Amino-6-chlor-4-nitrophenol | 0,013 | --- | --- | 0,013 |
| N,N-Bis(2-hydroxyethyl)-2-nitro-p-phenylendiamin | 0,006 | --- | --- | 0,006 |
| Silsoft AX | --- | 1,0 | --- | 1,0 |
| 1-Hydroxyethan-1,1-diphosphonsäure | ad pH 6,7 | ad pH 6,7 | --- | ad pH 6,7 |
| Monoethanolamin | 0,73 | 0,73 | ad pH 8,7 | 0,73 |
| Wasser dest. | ad 100 | ad 100 | ad 100 | ad 100 |

Eingesetzte Rohstoffe:

| | |
|---|---|
| Lorol C12-C18 techn. | $C_{12}$-$C_{18}$ Fettalkohole |
| Texapon NSO | Sodium Polyoxyethyelene Lauryl Ether Sulfat/ Sodium Polyoxyethylene Myristyl Ether Sulfat (27 %ige, wässrige Lsg.) |
| Dehydol LS 2 Deo N | Polyoxyethylen(2)dodecyl-Ether (INCI: Laureth-2) |
| Emulgade 1000 NI | Cetearyl Alkohol (80 - 100 %), Ceteareth-20 (10 - 20 %) |
| Carbomer | (Polyacrylsäure) |
| Silsoft AX | (INCI-Name: Bis-Cetearyl Amodimethicone [Momentive]) |

1.2. Apparative Bestimmung des Haarglanzes

Haartressen von 4 cm Breite und 15 cm Länge (Kerling, natural European Hair 6/0) wurden mit einer 0,3 %igen

Texapon-Lösung (pH 6-7, INCI-Name: SODIUM LAURETH SULFATE) vorgereinigt und getrocknet. Diese Strähne wurden in der einer Glanzapparatur vermessen.

Die zuvor hergestellten Formulierungen V1, V2, V3 und E wurden jeweils auf eine Haartresse aufgebracht (4 g Anwendungsmischung pro g Haar) und für 30 min bei 32 °C einwirken gelassen. Danach wurden die Tressen mit Leitungswasser ausgespült und getrocknet. Die getrockneten Haarsträhnen wurden ein zweites Mal in einer Glanzapparatur vermessen. Anschließend wurden die Haartressen dreimal mit einer wässrigen Shampoolösung gewaschen und getrocknet. Danach wurden die Strähnen ein drittes Mal in der Glanzapparatur vermessen.

Bei der Glanzapparatur handelt es sich um eine mit einer lichtabsorbierenden Auskleidung versehene Glanzkammer. Die zu vermessenden Haarsträhnen wurden auf einen Zylinder gespannt und in der Mitte der Glanzkammer positioniert. Oberhalb des Zylinders befindet sich als Beleuchtungsquelle eine stabförmige Gasentladungslampe, welche die auf dem Zylinder aufgespannte Haarsträhne durch eine kleine Blende beleuchtet. Mit einer Digitalkamera wurde die Glanzkurve jeder Strähne vermessen und bildanalytisch ausgewertet. Anschließend wurde der Glanzwert der Strähnen (L) nach der Formel nach Reich und Robbins (1) berechnet:

$$L_{Reich/Robbins} = \frac{S}{D * W_{\frac{1}{2}}}$$

L: Glanz

S: gerichtete Reflexion

D: diffuse Reflexion

W½: Halbwertsbreite der Glanzkurve

(1) C. Reich and C.C. Robbins, J. Soc. Cosmet. Chem. **44,** 221-234 (1993)

Hierbei wurden die nachfolgenden Glanzwerte erhalten. Je höher der Glanzwert ist, desto höher ist der Glanz.

| Glanzwerte | Haarglanz vor der Anwendung (Ausgangswert) | Haarglanz direkt nach der Anwendung | Haarglanz nach 3 Haarwäschen |
|---|---|---|---|
| V1 | 0,0114 | 0,0131 | 0,0133 |
| V2 | 0,0128 | 0,0126 | 0,0124 |
| V3 | 0,0120 | 0,0143 | 0,0128 |
| E | 0,0124 | 0,0178 | 0,0151 |

Es zeigt sich deutlich, dass mit den Haartressen, die mit der Formulierung E behandelt wurden, sowohl direkt nach der Anwendung als auch nach 3 Haarwäschen die höchsten Glanzwerte gemessen werden konnten.

Im Vergleich zu den Vergleichsformulierungen V1 bis V3 zeigt die erfindungsgemäße Formulierung E somit sowohl direkt nach der Anwendung als auch nach drei Haarwäschen den höchsten Glanz.

1.3. Bestimmung des Haarglanzes in Testsalonversuchen

Die Formulierungen V1 und E wurden bei jeweils 3 Probanden im Halbseitentest angewendet. Hierzu wurde die Formulierung V1 auf die eine Kopfhälfte, und die Formulierung E auf die anderen Kopfhälfte aufgetragen.

Nach der Anwendung wurden die Formulierungen abgespült und die Haare getrocknet. Anschließend wurde das Glanzergebnis jeder Formulierung visuell bewertet. Nach drei Haarwäschen wurde das Glanzergebnis ein zweites Mal visuell bewertet.

Bei allen drei Probanden wurde das folgende Ergebnis erhalten

| Haarglanz, visuell beurteilt | Haarglanz direkt nach der Anwendung | Haarglanz nach 3 Haarwäschen |
|---|---|---|
| V1 | Haarglanz vorhanden, tendentiell schwächer als bei E | kein Glanz |
| E | Haarglanz vorhanden, tendentiell stärker als bei V1 | noch stärkerer Glanz wahrnehmbar |

## Patentansprüche

1. Mittel zum Behandeln von keratinischen Fasern, insbesondere menschlichen Haaren, enthaltend in einem kosmetisch geeigneten Träger

   a) mindestens eine anorganische und/oder organische Säure
   b) mindestens einen direktziehenden Farbstoff und
   c) mindestens eine Verbindung der Formel (I)

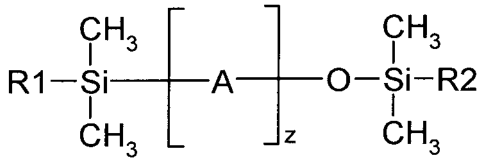

(I)

mit

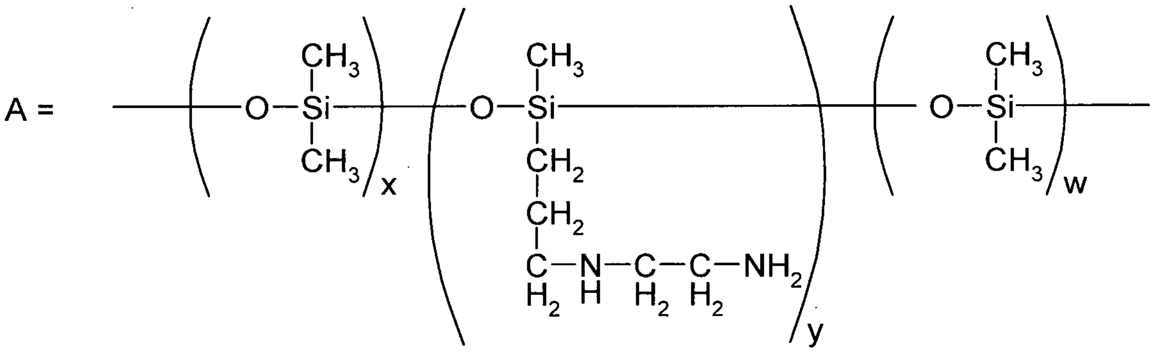

worin

   i. x und y unabhängig voneinander für Zahlen von 1 bis 100 stehen,
   ii. w für eine Zahl von 0 bis 100 steht,
   iii. z für eine Zahl von 1 bis 100 steht, wobei, falls z ≥ 2 ist, die jeweiligen Werte x, y und w in einem Strukturelement A jeweils unabhängig von vorangehenden Strukturelementen A gewählt werden können und
   iv. R1 und R2 unabhängig voneinander für eine lineare oder verzweigte, gesättigte, ungesättigte oder mehrfach ungesättigte $C_5$-$C_{20}$-Alkylgruppe stehen.

2. Mittel nach Anspruch 1, **dadurch gekennzeichnet, dass** es als anorganische und/oder organische Säure mindestens eine Säure ausgewählt aus Zitronensäure, Weinsäure, Äpfelsäure, Milchsäure, 1-Hydroxyethan-1,1-diphosphonsäure, 2,6-Dipicolinsäure und Benzoesäure enthält.

3. Mittel nach einem der Ansprüche 1 oder 2 **dadurch gekennzeichnet, dass** es bei einer Temperatur von 22 °C einen pH-Wert von 2 bis 7, bevorzugt von 3 bis 6,9, weiter bevorzugt von 4 bis 6,8 und insbesondere bevorzugt von 5 bis 6,7 besitzt.

4. Mittel nach einem der Ansprüche 1 bis 3, **dadurch gekennzeichnet, dass** es einen oder mehrere direktziehende Farbstoffe in einer Gesamtmenge von 0,0001 bis 5 Gew.-%, bevorzugt 0,001 bis 2,5 Gew.-%, weiter bevorzugt 0,01 bis 1 Gew.-% und insbesondere bevorzugt 0,02 bis 0,25 Gew.-%, bezogen auf das Gesamtgewicht des Mittels, enthält.

5. Mittel nach einem der Ansprüche 1 bis 4, **dadurch gekennzeichnet, dass** es als direktziehenden Farbstoff mindestens einen nichtionischen Nitrofarbstoff enthält.

6. Mittel nach einem der Ansprüche 1 bis 5, welches **dadurch gekennzeichnet ist, dass** es eine der folgenden Kombinationen an direktziehenden Farbstoffen enthält: HC Blue 12/ HC Yellow 2, HC Blue 12/ HC Yellow 4, HC Blue 12/ HC Yellow 5, HC Blue 12/ HC Yellow 6, HC Blue 12/ HC Yellow 12, HC Blue 12/ HC Orange 1, HC Blue 12/ HC Red 1, HC Blue 12/ HC Red 3, HC Blue 12/ HC Red 7, HC Blue 12/ HC Red 10, HC Blue 12/ HC Red 11, HC Blue 12/ HC Red 13, HC Blue 12/ HC Red BN, HC Blue 12/ HC Violet 1, HC Blue 12/1,4-Diamino-2-nitrobenzol, HC Blue 12/2-Amino-4-nitrophenol, HC Blue 12/1,4-Bis-(2-hydroxyethyl)amino-2-nitrobenzol, HC Blue 12/3-Nitro-4-(2-hydroxyethyl)aminophenol, HC Blue 12/4-[(2-Hydroxyethyl)amino]-3-nitro-1-methylbenzol, HC Blue 12/1-Amino-4-(2-hydroxyethyl)amino-5-chlor-2-nitrobenzol, HC Blue 12/1-Amino-4-(2-hydroxyethyl)amino-5-chlor-2-nitrobenzol, HC Blue 12/4-Amino-3-nitrophenol, HC Blue 12/4-[(3-Hydroxypropyl)amino]-3-nitrophenol, HC Blue 12/2-Amino-6-chloro-4-nitrophenol, HC Blue 12/4-Ethylamino-3-nitrobenzoesäure, HC Blue 12/2-Chlor-6-ethylamino-4-nitrophenol, HC Yellow 2/ HC Yellow 4, HC Yellow 2/ HC Yellow 5, HC Yellow 2/ HC Yellow 6, HC Yellow 2/ HC Yellow 12, HC Yellow 2/ HC Orange 1, HC Yellow 2/ HC Red 1, HC Yellow 2/ HC Red 3, HC Yellow 2/ HC Red 7, HC Yellow 2/ HC Red 10, HC Yellow 2/ HC Red 11, HC Yellow 2/ HC Red 13, HC Yellow 2/ HC Red BN, HC Yellow 2/HC Violet 1, HC Yellow 2 /HC Blue 2, HC Yellow 2/ HC Blue 11, HC Yellow 2/HC Blue 12, HC Yellow 2/1,4-Diamino-2-nitrobenzol, HC Yellow 2/2-Amino-4-nitrophenol, HC Yellow 2/1,4-Bis-(2-hydroxyethyl)amino-2-nitrobenzol, HC Yellow 2/3-Nitro-4-(2-hydroxyethyl)aminophenol, HC Yellow 2/4-[(2-Hydroxyethyl)amino]-3-nitro-1-methylbenzol, HC Yellow 2/1-Amino-4-(2-hydroxyethyl)amino-5-chlor-2-nitrobenzol, HC Yellow 2/4-Amino-3-nitrophenol, HC Yellow 2/4-[(3-Hydroxypropyl)amino]-3-nitrophenol, HC Yellow 2/2-Amino-6-chloro-4-nitrophenol, HC Yellow 2/4-Ethylamino-3-nitrobenzoesäure oder HC Yellow 2/2-Chlor-6-ethylamino-4-nitrophenol, 2-Amino-6-chloro-4-nitrophenol/HC Yellow 2, 2-Amino-6-chlor-4-nitrophenol/ HC Yellow 4, 2-Amino-6-chlor-4-nitrophenol/ HC Yellow 5, 2-Amino-6-chlor-4-nitrophenol/ HC Yellow 6, 2-Amino-6-chlor-4-nitrophenol/ HC Yellow 12, 2-Amino-6-chlor-4-nitrophenol/ HC Orange 1, 2-Amino-6-chlor-4-nitrophenol/ HC Red 1, 2-Amino-6-chlor-4-nitrophenol/ HC Red 3, 2-Amino-6-chlor-4-nitrophenol/ HC Red 7, 2-Amino-6-chlor-4-nitrophenol/ HC Red 10, 2-Amino-6-chlor-4-nitrophenol/ HC Red 11, 2-Amino-6-chlor-4-nitrophenol/ HC Red 13, 2-Amino-6-chlor-4-nitrophenol/ HC Red BN, 2-Amino-6-chlor-4-nitrophenol/ HC Blue 2, 2-Amino-6-chlor-4-nitrophenol/ HC Blue 11, 2-Amino-6-chlor-4-nitrophenol/ HC Blue 12, 2-Amino-6-chlor-4-nitrophenol/HC Violet 1, 2-Amino-6-chlor-4-nitrophenol/1,4-Diamino-2-nitrobenzol, 2-Amino-6-chlor-4-nitrophenol/2-Amino-4-nitrophenol, 2-Amino-6-chlor-4-nitrophenol/1,4-Bis-(2-hydroxyethyl)amino-2-nitrobenzol, 2-Amino-6-chlor-4-nitrophenol/3-Nitro-4-(2-hydroxyethyl)aminophenol, 2-Amino-6-chlor-4-nitrophenol/4-[(2-Hydroxyethyl)amino]-3-nitro-1-methylbenzol, 2-Amino-6-chlor-4-nitrophenol/1-Amino-4-(2-hydroxyethyl)amino-5-chlor-2-nitrobenzol, 2-Amino-6-chlor-4-nitrophenol/4-Amino-3-nitrophenol, 2-Amino-6-chlor-4-nitrophenol/4-[(3-Hydroxypropyl)amino]-3-nitrophenol, 2-Amino-6-chlor-4-nitrophenol/4-Ethylamino-3-nitrobenzoesäure oder 2-Amino-6-chlor-4-nitrophenol/2-Chlor-6-ethylamino-4-nitrophenol.

7. Mittel nach einem der Ansprüche 1 bis 6, **dadurch gekennzeichnet, dass** es eine Verbindung der Formel (I) enthält, in der die Substituenten R1 und R2 unabhängig voneinander für eine lineare $C_{14}$-$C_{20}$-Alkylkette, insbesondere bevorzugt für einen Vertreter aus der Gruppe $H_3C$-$(CH_2)_{13}$-, $H_3C$-$(CH_2)_{15}$-, $H_3C$-$(CH_2)_{17}$- und $H_3C$-$(CH_2)_{19}$-, stehen.

8. Mittel nach einem der Ansprüche 1 bis 7, **dadurch gekennzeichnet, dass** es die Verbindung bzw. die Verbindungen der Formel (I) in einer Gesamtmenge von 0,005 bis 5 Gew.-%, bevorzugt 0,1 bis 4,5 Gew.-%, besonders bevorzugt 1 bis 3 Gew.-% und insbesondere bevorzugt 1,5 bis 2,5 Gew.-%, bezogen auf das Gesamtgewicht des Mittels, enthält.

9. Mittel nach einem der Ansprüche 1 bis 8, **dadurch gekennzeichnet, dass** es ein oder mehrere Lösungsmittel aus der Gruppe 2-Butoxyethanol, Propylenglykolmonomethylether, Diethylengkykolmonomethylether, Diethylenglykolmonoethylether, Ethylenglykolmonobutylether, Ethylenglykolmonoethylether, Ethanol, Isopropanol, n-Propanol, n-Butanol, 1,3-Propandiol, Glycerin, 1-Methoxy-2-propanol, 1-Ethoxy-2-propandiol, 1,4-Butandiol, 1,3-Butandiol, 1,2-Hexandiol, Benzylalkohol, Phenoxyethanol, 2-Phenylethylalkohol, Methoxybutanol, n-Butylenglykol, Ethylencarbonat und Propylencarbonat in einer Gesamtmenge von 0,1 bis 15 Gew.-%, bevorzugt 1 bis 10 Gew.-%, weiter bevorzugt 1,5 bis 8 Gew.-% und insbesondere bevorzugt 2 bis 6 Gew.-%, bezogen auf das Gesamtgewicht des Mittels, enthält.

**10.** Mittel nach einem der Ansprüche 1 bis 9, **dadurch gekennzeichnet, dass** es eine oder mehrere Verbindungen der Formel (II)

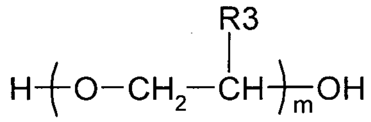

(II)

worin

R3 für ein Wasserstoffatom oder eine Methylgruppe steht und
m für eine Zahl von 2 bis 1000 steht,
in einem Gesamtgewicht von 0,1 bis 15 Gew.-%, bevorzugt 1 bis 10 Gew.-%, weiter bevorzugt 1,5 bis 8 Gew.-% und insbesondere bevorzugt 2 bis 6 Gew.-%, bezogen auf das Gesamtgewicht des Mittels, enthält.

**11.** Kosmetische, nicht-therapeutische Verwendung eines Mittels nach einem der Ansprüche 1 bis 10 zur Erzeugung von lang anhaltendem Glanz auf keratinischen Fasern.

**12.** Verfahren zur Erzeugung von lang anhaltendem Glanz auf keratinischen Fasern, insbesondere menschlichen Haaren, bei welchem ein Mittel nach einem der Ansprüche 1 bis 10 auf die keratinischen Fasern aufgetragen, dort für 30 Sekunden bis 45 Minuten belassen und anschließend mit Wasser oder einem tensidhaltigen Mittel wieder ausgespült wird.

**Claims**

**1.** An agent for treating keratin fibers, in particular human hair, containing in a cosmetically suitable carrier:

(a) at least one inorganic and/or organic acid
(b) at least one direct dye, and
(c) at least one compound of formula (I)

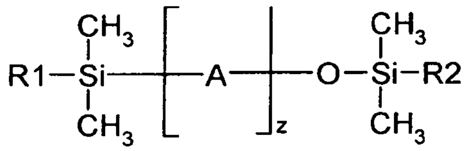

**(I)**

where

A = 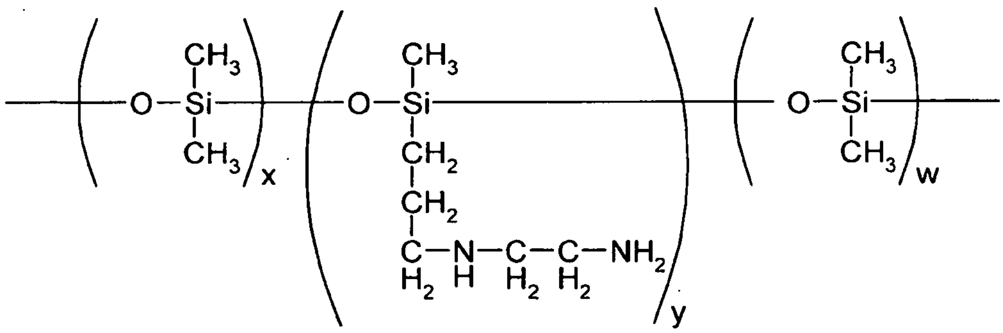

where

(i) x and y represent, independently of one another, numbers from 1 to 100,
(ii) w represents a number from 0 to 100,
(iii) z represents a number from 1 to 100, where, if $z \geq 2$, the respective values x, y, and w in a structural

element A may each be selected independently of preceding structural elements A, and

(iv) R1 and R2 represent, independently of one another, a linear or branched, saturated, unsaturated or polyunsaturated $C_5$-$C_{20}$ alkyl group.

2. The agent according to claim 1, **characterized in that** it contains, as an inorganic and/or organic acid, at least one acid selected from citric acid, tartaric acid, malic acid, lactic acid, 1-hydroxyethane-1,1-diphosphonic acid, 2,6-dipicolinic acid, and benzoic acid.

3. The agent according to one of claims 1 or 2, **characterized in that**, at a temperature of 22 °C, it has a pH of from 2 to 7, preferably of from 3 to 6.9, more preferably of from 4 to 6.8, and particularly preferably of from 5 to 6.7.

4. The agent according to one of claims 1 to 3, **characterized in that** it contains one or more direct dyes in a total amount of from 0.0001 to 5 wt.%, preferably 0.001 to 2.5 wt.%, more preferably 0.01 to 1 wt.%, and particularly preferably 0.02 to 0.25 wt.%, based on the total weight of the agent.

5. The agent according to one of claims 1 to 4, **characterized in that** it contains, as the direct dye, at least one non-ionic nitro dye.

6. The agent according to one of claims 1 to 5, **characterized in that** it contains one of the following combinations of direct dyes: HC Blue 12/HC Yellow 2, HC Blue 12/HC Yellow 4, HC Blue 12/HC Yellow 5, HC Blue 12/HC Yellow 6, HC Blue 12/HC Yellow 12, HC Blue 12/HC Orange 1, HC Blue 12/HC Red 1, HC Blue 12/HC Red 3, HC Blue 12/HC Red 7, HC Blue 12/HC Red 10, HC Blue 12/HC Red 11, HC Blue 12/HC Red 13, HC Blue 12/HC Red BN, HC Blue 12/HC Violet 1, HC Blue 12/1,4-diamino-2-nitrobenzene, HC Blue 12/2-amino-4-nitrophenol, HC Blue 12/1,4-bis-(2-hydroxyethyl)amino-2-nitrobenzene, HC Blue 12/3-nitro-4-(2-hydroxyethyl)aminophenol, HC Blue 12/4-[(2-hydroxyethyl)amino]-3-nitro-1-methylbenzene, HC Blue 12/1-amino-4-(2-hydroxyethyl)amino-5-chloro-2-nitrobenzene, HC Blue 12/1-amino-4-(2-hydroxyethyl)amino-5-chloro-2-nitrobenzene, HC Blue 12/4-amino-3-nitrophenol, HC Blue 12/4-[(3-hydroxypropyl)amino]-3-nitrophenol, HC Blue 12/2-amino-6-chloro-4-nitrophenol, HC Blue 12/4-ethylamino-3-nitrobenzoic acid, HC Blue 12/2-chloro-6-ethylamino-4-nitrophenol, HC Yellow 2/HC Yellow 4, HC Yellow 2/HC Yellow 5, HC Yellow 2/HC Yellow 6, HC Yellow 2/HC Yellow 12, HC Yellow 2/HC Orange 1, HC Yellow 2/HC Red 1, HC Yellow 2/HC Red 3, HC Yellow 2/HC Red 7, HC Yellow 2/HC Red 10, HC Yellow 2/HC Red 11, HC Yellow 2/HC Red 13, HC Yellow 2/HC Red BN, HC Yellow 2/HC Violet 1, HC Yellow 2/HC Blue 2, HC Yellow 2/HC Blue 11, HC Yellow 2/HC Blue 12, HC Yellow 2/1,4-diamino-2-nitrobenzene, HC Yellow 2/2-amino-4-nitrophenol, HC Yellow 2/1,4-bis-(2-hydroxyethyl)amino-2-nitrobenzene, HC Yellow 2/3-nitro-4-(2-hydroxyethyl)aminophenol, HC Yellow 2/4-[(2-hydroxyethyl)amino]-3-nitro-1-methylbenzene, HC Yellow 2/1-amino-4-(2-hydroxyethyl)amino-5-chloro-2-nitrobenzene, HC Yellow 2/4-amino-3-nitrophenol, HC Yellow 2/4-[(3-hydroxypropyl)amino]-3-nitrophenol, HC Yellow 2/2-amino-6-chloro-4-nitrophenol, HC Yellow 2/4-ethylamino-3-nitrobenzoic acid, or HC Yellow 2/2-chloro-6-ethylamino-4-nitrophenol, 2-amino-6-chloro-4-nitrophenol/HC Yellow 2, 2-amino-6-chloro-4-nitrophenol/HC Yellow 4, 2-amino-6-chloro-4-nitrophenol/HC Yellow 5, 2-amino-6-chloro-4-nitrophenol/HC Yellow 6, 2-amino-6-chloro-4-nitrophenol/HC Yellow 12, 2-amino-6-chloro-4-nitrophenol/HC Orange 1, 2-amino-6-chloro-4-nitrophenol/HC Red 1, 2-amino-6-chloro-4-nitrophenol/HC Red 3, 2-amino-6-chloro-4-nitrophenol/HC Red 7, 2-amino-6-chloro-4-nitrophenol/HC Red 10, 2-amino-6-chloro-4-nitrophenol/HC Red 11, 2-amino-6-chloro-4-nitrophenol/HC Red 13, 2-amino-6-chloro-4-nitrophenol/HC Red BN, 2-amino-6-chloro-4-nitrophenol/HC Blue 2, 2-amino-6-chloro-4-nitrophenol/HC Blue 11, 2-amino-6-chloro-4-nitrophenol/HC Blue 12, 2-amino-6-chloro-4-nitrophenol/HC Violet 1, 2-amino-6-chloro-4-nitrophenol/1,4-diamino-2-nitrobenzene, 2-amino-6-chloro-4-nitrophenol/2-amino-4-nitrophenol, 2-amino-6-chloro-4-nitrophenol/1,4-bis-(2-hydroxyethyl)amino-2-nitrobenzene, 2-amino-6-chloro-4-nitrophenol/3-nitro-4-(2-hydroxyethyl)aminophenol, 2-amino-6-chloro-4-nitrophenol/4-[(2-hydroxyethyl)amino]-3-nitro-1-methylbenzene, 2-amino-6-chloro-4-nitrophenol/1-amino-4-(2-hydroxyethyl)amino-5-chloro-2-nitrobenzene, 2-amino-6-chloro-4-nitrophenol/4-amino-3-nitrophenol, 2-amino-6-chloro-4-nitrophenol/4-[(3-hydroxypropyl)3mino]-3-nitrophenol, 2-amino-6-chloro-4-nitrophenol/4-ethylamino-3-nitrobenzoic acid, or 2-amino-6-chloro-4-nitrophenol/2-chloro-6-ethylamino-4-nitrophenol.

7. The agent according to one of claims 1 to 6, **characterized in that** it contains a compound of formula (I) in which the substituents R1 and R2 represent, independently of one another, a linear $C_{14}$-$C_{20}$ alkyl chain, particularly preferably a representative from the group comprising $H_3C-(CH_2)_{13}-$, $H_3C-(CH_2)_{15}-$, $H_3C-(CH_2)_{17}-$, and $H_3C-(CH_2)_{19}-$.

8. The agent according to one of claims 1 to 7, **characterized in that** it contains the compound(s) of formula (I) in a total amount of from 0.005 to 5 wt.%, preferably 0.1 to 4.5 wt.%, particularly preferably 1 to 3 wt.%, and particularly

preferably 1.5 to 2.5 wt.%, based on the total weight of the agent.

**9.** The agent according to one of claims 1 to 8, **characterized in that** it contains one or more solvents from the group comprising 2-butoxyethanol, propylene glycol monomethyl ether, diethylene glycol monomethyl ether, diethylene glycol monoethyl ether, ethylene glycol monobutyl ether, ethylene glycol monoethyl ether, ethanol, isopropanol, n-propanol, n-butanol, 1,3-propanediol, glycerin, 1-methoxy-2-propanol, 1-ethoxy-2-propanediol, 1,4-butanediol, 1,3-butanediol, 1,2-hexanediol, benzyl alcohol, phenoxyethanol, 2-phenylethyl alcohol, methoxybutanol, n-butylene glycol, ethylene carbonate, and propylene carbonate, in a total amount of from 0.1 to 15 wt.%, preferably 1 to 10 wt.%, more preferably 1.5 to 8 wt.%, and particularly preferably 2 to 6 wt.%, based on the total weight of the agent.

**10.** The agent according to one of claims 1 to 9, **characterized in that** it contains one or more compounds of formula (II)

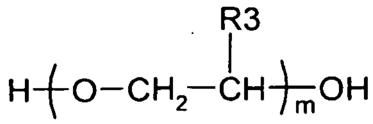

(II)

where

R3 represents a hydrogen atom or a methyl group, and
m represents a number from 2 to 1000,
in a total weight of from 0.1 to 15 wt.%, preferably 1 to 10 wt.%, more preferably 1.5 to 8 wt.%, and particularly preferably 2 to 6 wt.%, based on the total weight of the agent.

**11.** The cosmetic, non-therapeutic use of an agent according to one of claims 1 to 10 for producing a long-lasting shine on keratin fibers.

**12.** A method for producing a long-lasting shine on keratin fibers, in particular human hair, in which an agent according to one of claims 1 to 10 is applied to the keratin fibers, left there for 30 seconds to 45 minutes, and subsequently rinsed out again using water or a surfactant-containing agent.

**Revendications**

**1.** Produit de traitement de fibres de kératine, en particulier de cheveux humains, contenant, dans un vecteur cosmétiquement adéquat :

a) au moins un acide minéral et/ou organique
b) au moins un colorant direct, et
c) au moins un composé de formule (I)

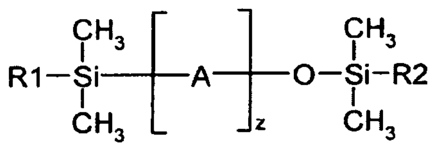

(I)

avec

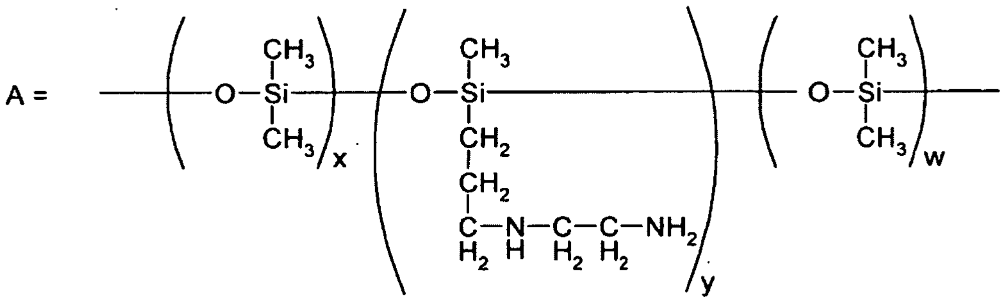

où

i) x et y représentent, indépendamment l'un de l'autre, un entier entre 1 et 100,
ii) w représente un entier entre 0 et 100,
iii) z représente un entier entre 1 et 100, où, si z $\geq$ 2, les valeurs respectives de x, y et w dans un élément de structure A peuvent être choisies indépendamment des éléments de structure A précédents,
et
iv) R1 et R2 représentent, indépendamment l'un de l'autre, un groupe $C_{5-20}$-alkyle linéaire ou ramifié, saturé, insaturé ou polyinsaturé.

2. Produit selon la revendication 1, **caractérisé en ce qu'**il contient comme acide minéral et/ou organique au moins un acide choisi parmi l'acide citrique, l'acide tartrique, l'acide malique, l'acide lactique, l'acide 1-hydroxyéthan-1,1-diphosphonique, l'acide 2,6-dipicolinique et l'acide benzoïque.

3. Produit selon une des revendications 1 ou 2, **caractérisé en ce qu'**il possède, à une température de 22°C, un pH entre 2 et 7, de préférence entre 3 et 6,9, plus préférentiellement entre 4 et 6,8 et en particulier entre 5 et 6,7.

4. Produit selon une des revendications 1 à 3, **caractérisé en ce qu'**il contient un ou plusieurs colorants directs à hauteur de 0,0001 à 5 % en poids, de préférence de 0,001 à 2,5 % en poids, plus préférentiellement de 0,01 à 1 % en poids et en particulier de 0,02 à 0,25 % en poids, rapporté au poids total du produit.

5. Produit selon une des revendications 1 à 4, **caractérisé en ce qu'**il contient comme colorant direct au moins un colorant nitro non-ionique.

6. Produit selon une des revendications 1 à 5, **caractérisé en ce qu'**il contient une des combinaisons suivantes de colorants directs : HC Blue 12 / HC Yellow 2, HC Blue 12 / HC Yellow 4, HC Blue 12 / HC Yellow 5, HC Blue 12 / HC Yellow 6, HC Blue 12 / HC Yellow 12, HC Blue 12 / HC Orange 1, HC Blue 12 / HC Red 1, HC Blue 12 / HC Red 3, HC Blue 12 / HC Red 7, HC Blue 12 / HC Red 10, HC Blue 12 / HC Red 11, HC Blue 12 / HC Red 13, HC Blue 12 / HC Red BN, HC Blue 12 / HC Violet 1, HC Blue 12/1,4-diamino-2-nitrobenzène, HC Blue 12/2-amino-4-nitrophénol, HC Blue 12/1,4-bis-(2-hydroxyéthyl)amino-2-nitrobenzène, HC Blue 12/3-nitro-4-(2-hydroxyéthyl)aminophénol, HC Blue 12 / 4-[(2-hydroxyéthyl)amino]-3-nitro-1-méthylbenzène, HC Blue 12/1-amino-4-(2-hydroxyéthyl)amino-5-chloro-2-nitrobenzène, HC Blue 12/1-amino-4-(2-hydroxyéthyl)amino-5-chloro-2-nitrobenzène, HC Blue 12/4-amino-3-nitrophénol, HC Blue 12/4-[(3-hydroxypropyl)amino]-3-nitrophénol, HC Blue 12/2-amino-6-chloro-4-nitrophénol, HC Blue 12 / acide 4-éthylamino-3-nitrobenzoïque, HC Blue 12 / 2-chloro-6-éthylamino-4-nitrophénol, HC Yellow 2 / HC Yellow 4, HC Yellow 2 / HC Yellow 5, HC Yellow 2 / HC Yellow 6, HC Yellow 2 / HC Yellow 12, HC Yellow 2 / HC Orange 1, HC Yellow 2 / HC Red 1, HC Yellow 2 / HC Red 3, HC Yellow 2 / HC Red 7, HC Yellow 2 / HC Red 10, HC Yellow 2 / HC Red 11, HC Yellow 2 / HC Red 13, HC Yellow 2 / HC Red BN, HC Yellow 2 / HC Violet 1, HC Yellow 2 / HC Blue 2, HC Yellow 2 / HC Blue 11, HC Yellow 2 / HC Blue 12, HC Yellow 2 / 1,4-diamino-2-nitrobenzène, HC Yellow 2 / 2-amino-4-nitrophénol, HC Yellow 2/1,4-bis-(2-hydroxyéthyl)amino-2-nitrobenzène, HC Yellow 2 / 3-nitro-4-(2-hydroxyéthyl)aminophénol, HC Yellow 2 / 4-[(2-hydroxyéthyl)amino]-3-nitro-1-méthylbenzène, HC Yellow 2 / 1-amino-4-(2-hydroxyéthyl)amino-5-chloro-2-nitrobenzène, HC Yellow 2 / 4-amino-3-nitrophénol, HC Yellow 2 / 4-[(3-hydroxypropyl)amino]-3-nitrophénol, HC Yellow 2 / 2-amino-6-chloro-4-nitrophénol, HC Yellow 2 / acide 4-éthylamino-3-nitrobenzoïque ou HC Yellow 2 / 2-chloro-6-éthylamino-4-nitrophénol, 2-amino-6-chloro-4-nitrophénol / HC Yellow 2, 2-amino-6-chloro-4-nitrophénol / HC Yellow 4, 2-amino-6-chloro-4-nitrophénol / HC Yellow 5, 2-amino-6-chloro-4-nitrophénol / HC Yellow 6, 2-amino-6-chloro-4-nitrophénol / HC Yellow 12, 2-amino-6-chloro-4-nitrophénol / HC Orange 1, 2-amino-6-chloro-4-nitrophénol / HC Red 1, 2-amino-6-chloro-4-nitrophénol / HC Red 3, 2-amino-6-chloro-4-nitrophénol / HC Red 7, 2-amino-6-chloro-4-nitrophénol / HC

Red 10, 2-amino-6-chloro-4-nitrophénol / HC Red 11, 2-amino-6-chloro-4-nitrophénol / HC Red 13, 2-amino-6-chloro-4-nitrophénol / HC Red BN, 2-amino-6-chloro-4-nitrophénol / HC Blue 2, 2-amino-6-chloro-4-nitrophénol / HC Blue 11, 2-amino-6-chloro-4-nitrophénol / HC Blue 12, 2-amino-6-chloro-4-nitrophénol / HC Violet 1, 2-amino-6-chloro-4-nitrophénol / 1,4-diamino-2-nitrobenzène, 2-amino-6-chloro-4-nitrophénol / 2-amino-4-nitrophénol, 2-amino-6-chloro-4-nitrophénol / 1,4-bis-(2-hydroxyéthyl)amino-2-nitrobenzène, 2-amino-6-chloro-4-nitrophénol / 3-nitro-4-(2-hydroxyéthyl)aminophénol, 2-amino-6-chloro-4-nitrophénol / 4-[(2-hydroxyéthyl)amino]-3-nitro-1-méthylbenzène, 2-amino-6-chloro-4-nitrophénol / 1-amino-4-(2-hydroxyéthyl)amino-5-chloro-2-nitrobenzène, 2-amino-6-chloro-4-nitrophénol / 4-amino-3-nitrophénol, 2-amino-6-chloro-4-nitrophénol / 4-[(3-hydroxypropyl)amino]-3-nitrophénol, 2-amino-6-chloro-4-nitrophénol / acide 4-éthylamino-3-nitrobenzoïque ou 2-amino-6-chloro-4-nitrophénol / 2-chloro-6-éthylamino-4-nitrophénol.

7. Produit selon une des revendications 1 à 6, **caractérisé en ce qu'**il contient un composé de formule (I), dans lequel les substituants R1 et R2 représentent, indépendamment l'un de l'autre, une chaîne $C_{14-20}$-alkyle linéaire, en particulier un représentant du groupe $H_3C-(CH_2)_{13}-$, $H_3C-(CH_2)_{15}-$, $H_3C-(CH_2)_{17}-$ et $H_3C-(CH_2)_{19}-$.

8. Produit selon une des revendications 1 à 7, **caractérisé en ce qu'**il contient le ou les composés de formule (I) à hauteur de 0,005 à 5 % en poids, de préférence de 0,1 à 4,5 % en poids, plus préférentiellement de 1 à 3 % en poids et en particulier de 1,5 à 2,5 % en poids, rapporté au poids total du produit.

9. Produit selon une des revendications 1 à 8, **caractérisé en ce qu'**il contient un ou plusieurs solvants issus du groupe 2-butoxyéthanol, monométhyléther de propylène glycol, monométhyléther de diéthylène glycol, monoéthyléther de diéthylène glycol, monobutyléther d'éthylène glycol, monoéthyléther d'éthylène glycol, éthanol, isopropanol, n-butanol, 1,3-propandiol, glycérine, 1-méthoxy2-propanol, 1-éthoxy2-propanol, 1,4-butandiol, 1,3-butandiol, 1,2-hexandiol, alcool benzylique, phénoxyéthanol, 2-phényléthanol, méthoxybutanol, n-butylène glycol, carbonate d'éthylène et carbonate de propylène, à hauteur de 0,1 à 15 % en poids, de préférence de 1 à 10 % en poids, plus préférentiellement de 1,5 à 8 % en poids et en particulier de 2 à 6 % en poids, rapporté au poids total du produit.

10. Produit selon une des revendications 1 à 9, **caractérisé en ce qu'**il contient un ou plusieurs composés de formule (II)

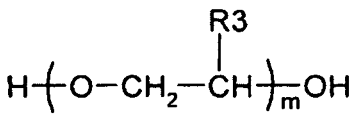

(II)

où

R3 représente un atome d'hydrogène ou un groupe méthyle, et
m représente un entier entre 2 et 1000,
à hauteur de 0,1 à 15 % en poids, de préférence de 1 à 10 % en poids, plus préférentiellement de 1,5 à 8 % en poids et en particulier de 2 à 6 % en poids, rapporté au poids total du produit.

11. Utilisation cosmétique non-thérapeutique d'un produit selon une des revendications 1 à 10 pour la création d'un éclat durable sur des fibres de kératine.

12. Procédé de création d'un éclat durable sur des fibres de kératine, en particulier sur des cheveux humains, dans lequel on applique un produit selon une des revendications 1 à 10 sur les fibres de kératine, on l'y laisse de 30 secondes à 45 minutes, puis on rince à l'eau ou avec un produit contenant des tensioactifs.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 1997038667 A
- DE 19757508
- DE 102011089686
- US 4820308 A
- US 2005255075 A
- US 20090074699 A1
- WO 2012066722 A1
- US 20030147841 A1